# EUROPÄISCHE PATENTSCHRIFT

(11) **EP 2 482 910 B1**
(45) Veröffentlichungstag und Bekanntmachung des Hinweises auf die Patenterteilung: **08.07.2020**
(21) Anmeldenummer: 10760900.0
(22) Anmeldetag: 29.09.2010
(51) Int. Cl.: A61M 25/09

(54) **FÜHRUNGSDRAHT**
GUIDE WIRE
FIL DE GUIDAGE

(30) Priorität: 30.09.2009 CH 15062009
(43) Veröffentlichungstag der Anmeldung: 08.08.2012
(60) Teilanmeldung: 14001967.0
(73) Patentinhaber: SIS Medical AG, 8500 Frauenfeld (CH)
(72) Erfinder: SCHWAGER, Michael, CH-8404 Winterthur (CH)
(74) Vertreter: Keller & Partner Patentanwälte AG
(86) Internationale Anmeldenummer: PCT/CH2010/000234
(87) Internationale Veröffentlichungsnummer: WO 2011/038522

(56) Entgegenhaltungen:
- EP-A2- 0 313 836
- EP-A2- 0 343 509
- EP-A2- 0 806 220
- WO-A1-96/13295
- WO-A1-97/11735
- WO-A1-97/49447
- WO-A2-93/04722
- US-A- 4 964 409
- US-A- 5 211 636
- US-A- 5 322 508
- US-A1- 2007 213 689

## Beschreibung

### Technisches Gebiet

Die Erfindung betrifft einen Führungsdraht für einen Katheter, welcher zum Einbringen und/oder Entnehmen von Fluiden in menschlichen und/oder tierischen Hohlorganen, insbesondere in Blutgefässen, ausgelegt ist, umfassend einen länglichen Hohlschaft mit einem Lumen zum Zu- und/oder Abführen des Fluids, sowie eine koaxial an einem distalen Ende des Hohlschafts anschliessende Einführhilfe in Form einer flexiblen Drahtwendel mit einer an einem distalen Ende angeordneten Führungsdrahtspitze, wobei zur Steuerung einer Flexibilität des Führungsdrahts im Lumen des Hohlschafts ein Kerndraht angeordnet ist, welcher sich aus dem Lumen heraus in einer longitudinalen Richtung durch die Drahtwendel hindurch bis zur Führungsdrahtspitze hin erstreckt. Des Weiteren beschreibt die Offenbarung ein Verfahren zur diagnostischen und/oder therapeutischen Behandlung von Gefässen und/oder Tumoren sowie eine Verwendung eines Führungsdrahts für diagnostische und/oder therapeutische Zwecke am menschlichen und/oder tierischen Körper.

### Stand der Technik

Führungsdrähte dienen üblicherweise als Hilfsmittel zum Einführen von Kathetern in menschliche und/oder tierische Hohlorgane. Die Führungsdrähte werden dabei vor dem Einführen des Katheters in das Hohlorgan eingeschoben und an einer gewünschten Stelle positioniert. Hierfür müssen die Führungsdrähte am distalen Ende ausreichend flexibel und dünn sein, um beispielsweise auch engen und stark gekrümmten Gefässverläufen zu folgen. Gleichzeitig müssen die Führungsdrähte aber auch eine ausreichende Steifigkeit aufweisen, damit sie vom proximalen Ende her vorgeschoben werden können.

Bekannt sind auch hohle Führungsdrähte, welche z. B. zum Einbringen und/oder Entnehmen von Flüssigkeit sowie als Druckmesssonden in einem Hohlorgan verwendet werden. Derartige Führungsdrähte verfügen im proximalen Teil beispielsweise über ein hohles Stahlröhrchen, welches am distalen Ende über eine relativ flexible Schraubenfeder mit einer Führungsdrahtspitze verfügt. Um die Steifigkeit des Führungsdrahts zu kontrollieren, wird am distalen Ende des Stahlröhrchens beispielsweise ein Kerndraht angebracht, welcher sich durch die Schraubenfeder bis zur Führungsdrahtspitze hin erstreckt. Der Kerndraht ist zudem im Bereich des proximalen Endes der Schraubenfeder rundum mit dieser verschweisst, um ein definiertes Schieben des Führungsdrahts zu ermöglichen.

Um ein Fluid durch den Führungsdraht zu- und/oder abzuführen, sind im Stahlröhrchen hinter der Drahtwendel eine oder mehrere seitliche Öffnungen eingebracht. Dadurch kann ein Fluid durch den hohlen Führungsdraht und die seitlichen Öffnungen in das Hohlorgan zugeführt werden oder es wird ein Fluid durch die seitlichen Öffnungen und den Führungsdraht aus dem Hohlorgan entnommen. Ein hohler Führungsdraht, welcher als Druckmesssonde eingesetzt wird, ist z. B. in der WO 97/32518 (Scimed Life Systems Inc.) beschrieben.

Aus der US 2007 213 689 A1 (Johnson & Johnson) ist ein lenkbarer Führungsdraht für Infusionen bekannt. Das führbare Ende wird durch eine Drahtwendel gebildet. Um die Infusionsflüssigkeit in den Bereich der Drahtwendel leiten zu können, ist in dem Führungsdraht ein eigenständiger Infusionsschlauch untergebracht, welcher sich vom proximalen Ende bis in den Bereich der Drahtwendel erstreckt, wo die therapeutische Flüssigkeit durch die Lücken zwischen den Windungen der Drahtwendel aus dem Führungsdraht herausfliessen kann. Die Drahtwendel ist zwischen einem inneren und äusseren Mantel aus Kunststoff untergebracht. Nachteilig ist hier, dass ein separater Infusionsschlauch im Führungsdraht untergebracht werden muss und dass der Austritt der Flüssigkeit im Wendelbereich nicht kontrolliert werden kann.

Die WO 97/11735 A1 (Interventional Innovations) offenbart einen Führungsdraht, dessen distaler Teil einen kleineren Durchmesser aufweist als dessen proximaler Teil. Eine offene Drahtwendel aus Wolfram oder Platin ist am distalen Teil des Führungsdrahts befestigt, um die distale Spitze des Führungsdrahts zum Schutz von Gewebe weicher zu gestalten. Ein aus einem distalen Ende des Hohldrahts heraus und in die Drahtspirale hinein ragendes Stabelement mit einer Nut für den Flüssigkeitsaustausch versehen. Die offene Drahtwendel ist nicht für den Flüssigkeitstransport geeignet, so dass die Flüssigkeit nur genau dort abgegeben werden kann, wo die Nut aus dem Führungsdraht hervortritt.

Aus der US 5 322 508 A (Cordis Corporation) ist ein weiterer Führungsdraht bekannt, welcher ein Flüssigkeitstransportsystem aufweist. Auf einen Kerndraht ist eine Drahtwendel aufgewickelt, auf deren Aussenseite ein Kunststoffmantel aufgebracht ist. Die Flüssigkeit wird in den Zwischenräumen der Wendel schraubenlinienartig nach vorn transportiert. Am Ende der Kunststoffummantelung kann die Flüssigkeit nach aussen treten. Nachteilig ist, dass durch die schraubenlinienartige Führung ein sehr langer und enger Kanal mit entsprechend erhöhtem Fliesswiderstand gegeben ist.

Die US 5 211 636 A (Lake Region Manufacturing) schlägt einen Führungsdraht vor, der aus einer äusseren und einer inneren Schraubenfeder besteht, in deren Zentrum ein Kerndraht angeordnet ist. Die äussere Schraubenfeder hat die Struktur eines schraubenlinienförmigen Flachbandes, welches aus vier parallel nebeneinander verlaufenden und miteinander verbundenen Wendeldrähten gebildet ist. Die innere Schraubenfeder ist durch einen Einzeldraht gebildet, wobei zwischen benachbarten Windungen ein Abstand besteht. Die Wendeln sind gegenläufig geführt, so dass der Führungsdraht in der Lage ist, ein Drehmoment zu übertragen. Der Hauptteil der Schraubenfeder ist dicht gewickelt, um eine maximale Kontrolle des Führungsdrahts zu erreichen. Die Schraubenfeder ist am distalen Ende beabstandet gewickelt, wobei die Abstände zwischen den Wicklungen als Infusionsöffnungen dienen. An der Innenseite der inneren Wendel oder zwischen innerer und äusserer Wendel ist eine Membran vorgesehen, damit das zentrale Lumen, in welchem sich auch der Kerndraht befindet, als Kanal für die Infusions-Flüssigkeit benutzt werden kann.

WO 97/49447 A1 (Therox Inc. et al.) beschreibt Führungsdrähte zur in-situ Abgabe von Fluiden an vaskulären Stellen, welche für hohe Drücke geeignet sind. Der Führungsdraht weist ein proximales rohrförmiges Segment, eine optionale Zwischenregion zur Regulierung von Druckverhältnissen und ein distales Segment auf, welches die Flüssigkeit aus dem Perfusionsführungsdraht entlässt. In einer Ausführungsform des distalen Endes umfasst das distale Segment einen Kerndraht, welcher an seinem distalen Ende von einer Drahtwendel umgeben ist und aus einem dünnwandigen Mantel hervortritt. Der Mantel umfasst ein Polymerrohr, wobei das distale Ende des Polymerrohres offen oder geschlossen sein kann. Ist das Rohr geschlossen, so sind Kanäle zum Flüssigkeitsdurchtritt vorgesehen.

Die heute bekannten hohlen Führungsdrähte zum Zu- und/oder Abführen eines Fluids vermögen jedoch nicht vollständig zu befriedigen.

Es besteht daher nach wie vor Bedarf nach einem verbesserten leicht einführbaren Führungsdraht zum Zu- und/oder Abführen eines Fluids.

### Darstellung der Erfindung

Aufgabe der Erfindung ist es, ein dem eingangs genannten technischen Gebiet zugehörenden leicht einführbaren Führungsdraht zu schaffen, welcher ein präziseres Einbringen und/oder Entnehmen von Fluiden in menschlichen und/oder tierischen Hohlorganen ermöglicht.

Die Lösung der Aufgabe ist durch die Merkmale des Anspruchs 1 definiert. Gemäss der Erfindung kommuniziert ein in einer proximalen Richtung an die Führungsdrahtspitze angrenzender distaler Innenbereich der Drahtwendel über einen neben dem Kerndraht ausgebildeten Fluidkanal mit dem Lumen des Hohlschafts und verfügt zudem über wenigstens eine nach aussen offene Durchlassöffnung für das ein- und/oder auszubringende Fluid.

Unter einer Drahtwendel wird in diesem Zusammenhang insbesondere eine hohlzylindrische Struktur verstanden, welche durch einen schraubenförmig um eine Längsachse gewundenen Draht gebildet wird. Die Drahtwendel besteht dabei insbesondere aus einem Metalldraht, z. B. aus Platin. Der Draht der Drahtwendel muss jedoch nicht zwingend aus Metall bestehen. Grundsätzlich ist es auch denkbar einen Kunststoffdraht vorzusehen.

Der Kerndraht ist insbesondere aus Metall, beispielsweise aus Stahl, gefertigt. Es sind prinzipiell aber auch andere Materialien für den Kerndraht verwendbar, z.B. hochfeste Kunststoffe und/oder Kompositmaterialien.

Durch die erfindungsgemässe Anordnung der Durchlassöffnung in einem an die Führungsdrahtspitze angrenzenden distalen Innenbereich der Drahtwendel liegt die Durchlassöffnung in einem Bereich in unmittelbarer Nähe zur Führungsdrahtspitze. Im Zusammenspiel mit dem Fluidkanal zwischen dem distalen Innenbereich und dem Lumen des Hohlschafts kann ein Fluid somit direkt in einem Bereich der Führungsdrahtspitze aus dem Führungsdraht in ein Hohlorgan eingebracht und/oder aus diesem entnommen werden. Bevorzugt ist die wenigstens eine Öffnung des distalen Innenbereichs der Drahtwendel in proximaler Richtung unmittelbar an die Führungsdrahtspitze angrenzend angeordnet.

Da sich die Position der Führungsdrahtspitze in einem Hohlorgan im Allgemeinen relativ präzise bestimmen lässt, z. B. durch bekannte bildgebende Verfahren, beispielsweise unter Verwendung von Röntgenstrahlung, ist aufgrund der Nähe der wenigstens einen Durchlassöffnung zur Führungsdrahtspitze auch die Position der wenigstens einen Durchlassöffnung sehr genau bestimmbar. Speziell im Vergleich mit bekannten Führungsdrähten, bei welchen die Durchlassöffnungen in proximaler Richtung hinter der Drahtwendel angeordnet sind, kann die Position der Durchlassöffnung beim erfindungsgemässen Führungsdraht daher präziser bestimmt werden.

Aufgrund des aus dem Lumen durch die Drahtwendel bis zur Führungsdrahtspitze verlaufenden Kerndrahts ist die Elastizität des Führungsdrahts in seinen distalen Bereichen zudem sehr genau einstellbar, was die Einführbarkeit und Steuerbarkeit des Führungsdrahts in einem Hohlorgan zusätzlich verbessert. Konkret ist es zum Beispiel möglich, Elastizitätssprünge im Bereich des Übergangs zwischen Hohlschaft und Drahtwendel durch den Kerndraht auszugleichen, was beispielsweise einem Abknicken des Führungsdrahts beim Einführen entgegenwirkt. Ebenso kann die Elastizität der üblicherweise überelastischen Drahtwendel durch den Kerndraht reduziert und an die spezifischen Anforderungen angepasst werden. Dadurch ist es insbesondere möglich, Drahtwendel mit besonders geringen Drahtdurchmessern einzusetzen, was insbesondere Platz spart. Der Führungsdraht oder die Führungsdrahtspitze kann damit gezielter und präziser an eine gewünschte Stelle im Hohlorgan bewegt oder manövriert werden. Wie sich gezeigt hat, wird im Zusammenspiel mit der erfindungsgemässen Anordnung der Durchlassöffnung dadurch die Präzision beim Einbringen und/oder Entnehmen von Fluiden in menschlichen und/oder tierischen Hohlorganen in überraschendem Masse gesteigert.

Zudem erlaubt die erfindungsgemässe Lösung eine Platz sparende und kompakte Bauweise, da der Fluidkanal zwischen Lumen und distalem Innenbereich der Drahtwendel vollständig innerhalb des Lumens und der Drahtwendel verlaufen kann. Bevorzugt wird im Lumen des Hohlschafts und/oder im Innenbereich der Drahtwendel neben dem Kerndraht ein Freiraum ausgebildet, welcher als Fluidkanal fungiert. In einer besonders vorteilhaften Ausführungsform verfügt der Hohlschaft über eine stirnseitige Öffnung an seinem distalen Ende, welche über eine stirnseitige Öffnung der Drahtwendel an ihrem proximalen Ende kommuniziert. Mit Vorteil sind die Querschnittsflächen der beiden stirnseitigen Öffnungen am Hohlschaft und an der Drahtwendel dabei grösser als eine in diesem Bereich vorliegende Querschnittsfläche des Kerndrahts. Damit bleibt neben dem Kerndraht ein Freiraum, welcher eine Durchleitung des Fluids ermöglicht.

Bei einer derartigen Konstruktion können die erfindungsgemässen Führungsdrähte mit einer über ihre gesamte Länge im Wesentlichen konstanten oder zur Führungsdrahtspitze hin kontinuierlich abnehmenden Querschnittsfläche ausgebildet werden, was wiederum der Einführbarkeit des Führungsdrahts zu Gute kommt.

Das distale Ende des Hohlschafts und das proximale Ende der Drahtwendel sind z. B. miteinander verschweisst und/oder verklebt. Insbesondere kann dabei ein proximales Ende der Drahtwendel stirnseitig an einem distalen Ende des Hohlschafts anliegen. Es ist aber auch möglich, das proximale Ende der Drahtwendel auf das distale Ende des Hohlschafts aufzuschieben, so dass das proximale Ende der Drahtwendel das distale Ende des Hohlschafts umgibt. Auch vorteilhaft ist eine Anordnung bei welcher das proximale Ende der Drahtwendel in das distale Ende des Hohlschafts hinein geschoben wird. In diesem Fall umgibt das distale Ende des Hohlschafts das proximale Ende der Drahtwendel. Insgesamt sind die erfindungsgemässen Führungsdrähte leicht in menschliche und/oder tierische Hohlorgane einführbar und ermöglichen zudem ein hochpräzises und gezieltes Einbringen und/oder Entnehmen von Fluiden an definierten Stellen in menschlichen und/oder tierischen Hohlorganen.

Bevorzugt liegt in einem Bereich eines proximalen Endes der Drahtwendel eine fluiddurchlässige Verbindung zwischen Drahtwendel und Kerndraht vor, so dass die Drahtwendel gegenüber dem Kerndraht mechanisch fixiert ist und der distale Innenbereich der Drahtwendel mit einem proximal der fluiddurchlässigen Verbindung vorliegenden proximalen Innenbereich der Drahtwendel kommuniziert. Dadurch wird insbesondere ein noch präziseres Schieben des Führungsdrahts in einem Hohlorgan möglich, da Stauchungen und/oder Dehnungen der Drahtwendel in longitudinaler Richtung nahezu vollständig verhindert werden, ohne dabei die transversale Flexibilität des Führungsdrahts wesentlich zu beeinträchtigen. Da die Verbindung zwischen Drahtwendel und Kerndraht insbesondere fluiddurchlässig ist, kommuniziert der proximal der Verbindung liegende proximalen Innenbereich dennoch weiterhin mit dem distal der Verbindung liegenden distalen Innenbereich der Drahtwendel. Dadurch lässt sich auch bei einer stabilen mechanischen Verbindung zwischen Kerndraht und Drahtwendel in einfacher Art und Weise ein Fluidkanal in longitudinaler Richtung durch die Drahtwendel realisieren.

Falls notwendig, z. B. bei sehr langen Drahtwendeln, können mehrere und in longitudinaler Richtung beabstandete fluiddurchlässige Verbindungen zwischen Drahtwendel und Kerndraht vorliegen. Grundsätzlich ist es aber auch denkbar auf eine fluiddurchlässige Verbindung zu verzichten. Allerdings kann dies bei sehr engen Hohlorganen, welche einen grossen Widerstand beim Bewegen des Führungsdrahts erzeugen, unter Umständen nachteilig sein, da die Drahtwendel beim Bewegen möglicherweise stark gestaucht und/oder gedehnt wird.

Im Besonderen liegt die fluiddurchlässige Verbindung als eine bezüglich einer Längsmittelachse der Drahtwendel asymmetrisch und/oder einseitig ausgebildete stoffschlüssige Verbindung, insbesondere eine Lotverbindung, zwischen Drahtwendel und Kerndraht vor. Dadurch bleibt im Bereich der fluiddurchlässigen Verbindung ein seitlich des Kerndrahts liegender kanalartiger Durchlass für das zu- und/oder abzuführende Fluid frei. Zudem werden bei einer derartig ausgebildeten fluiddurchlässigen Verbindung keine zusätzlichen Elemente benötigt, was im Besonderen den Platzbedarf reduziert und eine entsprechend kompaktere Bauweise des Führungsdrahts ermöglicht.

Die stoffschlüssige Verbindung ist insbesondere eine Lotverbindung. Es ist jedoch auch möglich eine Klebverbindung und/oder eine Verschweissung vorzusehen. Insbesondere können auch mehrere getrennte Verbindungsstege, z. B. aus einem Lot und/oder einem Klebstoff, welche beispielsweise in radialer Richtung zwischen Kerndraht und Drahtwendel verlaufen. Die Verbindungsstege bilden dann eine fluiddurchlässige Verbindung, da die Freiräume zwischen den Verbindungsstegen einen Fluiddurchlass bilden.

In einer weiteren bevorzugten Ausführungsform ist die fluiddurchlässige Verbindung als stoffschlüssige Verbindung, insbesondere als Lotverbindung, ausgebildet, wobei ein parallel zum Kerndraht verlaufendes und in die stoffschlüssige Verbindung eingebettetes Röhrchen als kanalartiger Durchlass für das zu- und/oder abzuführende Fluid vorliegt. Die stoffschlüssige Verbindung kann z. B. als Lot- und/oder Klebverbindung ausgeführt sein. Aufgrund des Röhrchens verfügt die fluiddurchlässige Verbindung einerseits über eine relativ genau definierte Durchlassöffnung. Andererseits hat sich gezeigt, dass ein Röhrchen relativ einfach in der stoffschlüssigen Verbindung eingebettet werden kann, was die Herstellung des Führungsdrahts vereinfacht. Der Kerndraht mit dem daneben angeordneten Röhrchen kann in diesem Fall rundum stoffschlüssig mit der Drahtwendel verbunden werden, was meist einfacher ist, als den Kerndraht einseitig mit der Drahtwendel zu verbinden. Zudem wird eine stabilere Verbindung zwischen Kerndraht und Drahtwendel gebildet.

Der Kerndraht ist mit Vorteil in einer proximalen Richtung vom proximalen Ende der Drahtwendel beabstandet im Lumen fixiert, und insbesondere exzentrisch bezüglich einer longitudinalen Achse des Lumens angeordnet. Dadurch verläuft der Kerndraht vom proximalen Ende der Drahtwendel vollständig durch diese hindurch bis zum distalen Ende oder der Führungsdrahtspitze. Wie sich herausgestellt hat, ist eine derartige Anordnung besonders vorteilhaft, da der üblicherweise vorhandene Elastizitätssprung zwischen dem Hohlschaft und der Drahtwendel durch den Kerndraht bestmöglich kompensiert werden kann. Eine exzentrische Anordnung des Kerndrahts an einer Begrenzungsfläche des Lumens anliegend vereinfacht zudem die Herstellung des Führungsdrahts. Bestehen der Kerndraht und der Hohlschaft bzw. die Begrenzungsfläche des Lumens aus ähnlichen Materialien, z. B. Metallen, kann der Kerndraht beispielsweise direkt an die Begrenzungsfläche des Lumens angeschweisst werden.

Es ist aber auch möglich, einen in die Drahtwendel hinein ragenden Hohlschaft vorzusehen und den Kerndraht in distaler Richtung vom proximalen Ende des Drahtwendel beabstandet anzubringen. Dies kann für spezielle Anwendungen unter Umständen vorteilhaft sein, obschon in diesem Fall ein relativ grosser Elastizitätssprung zwischen Hohlschaft und Drahtwendel auftreten kann.

Bevorzugt verfügen das Lumen und/oder die Drahtwendel über eine kreisförmige Querschnittsfläche, wobei insbesondere ein maximaler Aussendurchmesser des Kerndrahts geringer ist als ein minimaler Innendurchmesser des Lumens und/oder geringer als ein minimaler Innendurchmesser der Drahtwendel. Besonders bevorzugt ist der minimale Innendurchmesser des Lumens und/oder der minimale Innendurchmesser der Drahtwendel 3 - 4 Mal grösser, als der Aussendurchmesser des Kerndrahts im Bereich der Drahtwendel. Dadurch bleibt neben dem Kerndraht entlang der gesamten Länge des Kerndrahts im Lumen und/oder in der Drahtwendel ein Freiraum, welcher als (parallel zur Längsachse des Führungsdrahts verlaufender) Fluidkanal für das zu- und/oder abzuführende Fluid dienen kann. Dies stellt eine besonders einfache Massnahme zur Ausbildung eines Fluidkanals dar, was herstellungstechnisch vorteilhaft ist. Des Weiteren wird dadurch eine besonders kompakte Bauweise des Führungsdrahts ermöglicht, was wiederum der Einführbarkeit und der präzisen Bewegung des Führungsdrahts in einem Hohlorgan zu Gute kommt.

Eine derartige Ausgestaltung ist jedoch nicht zwingend. Prinzipiell kann der Kerndraht das Lumen und/oder die Drahtwendel auch vollständig ausfüllen. Insbesondere können im Innern des Kerndrahts z. B. Durchlasskanäle angeordnet sein, über welche das zu- und/oder abzuführende Fluid zwischen Lumen und distalem Innenbereich der Drahtwendel geleitet werden kann. Ebenso ist es möglich, im Hohlschaft und/oder aussen am Hohlschaft wenigstens ein weiteres in longitudinaler Richtung verlaufendes Lumen als Fluidkanal zum Durchleiten des zu- und/oder abzuführenden Fluids anzubringen, sofern dies als zweckmässig erachtet wird.

Der Kerndraht, der Hohlschaft, das Lumen und/oder die Drahtwendel verfügen nicht zwingend über einen kreisrunden Querschnitt. Die genannten Elemente des Führungsdrahts können beispielsweise auch ovale Querschnitte aufweisen, sofern dies zweckdienlich erscheint. In diesen Fällen ist unter dem Durchmesser insbesondere eine maximale Ausdehnung des jeweiligen Elements in einer transversalen Richtung senkrecht zur longitudinalen Richtung des Führungsdrahts zu verstehen.

Zur Erzeugung der wenigstens einen Öffnung sind erfindungsgemäss wenigstens zwei benachbarte Windungen der Drahtwendel voneinander beabstandet, insbesondere berührungslos, angeordnet. Zwischen den wenigstens zwei benachbarten Windungen liegt dabei mit Vorteil ein Abstand vor, welcher 0.1 - 0.5 Mal einem Drahtdurchmesser der Drahtwendel entspricht. Eine derartige Öffnung mündet insbesondere seitlich oder in transversaler Richtung aus der Drahtwendel. Insbesondere bei einer Drahtwendel aus einem runden Draht, ist die Öffnung im Wesentlichen vollständig kantenfrei. Dies garantiert im Besonderen eine gute Einführbarkeit und Positionierbarkeit des Führungsdrahts in einem Hohlorgan, da die Gefahr des Verhakens und/oder Hängenbleibens der Öffnung im Hohlorgan minimal ist. Zudem lässt sich die Grösse oder die Fläche der Öffnung in einfacher Weise über den Abstand zwischen den die Öffnung bildenden benachbarten Windungen und/oder die Anzahl der beabstandeten Windungen einstellen. Damit kann die Öffnung in weiten Bereichen an unterschiedlichste Anforderungen angepasst werden. Zudem kann der Führungsdraht besonders kompakt ausgebildet sein, da keine zusätzlichen Elemente für die Öffnung angeordnet werden müssen. Ein Abstand von 0.1 - 0.5 Mal dem Drahtdurchmesser zwischen den benachbarten Windungen einer Öffnung gewährleistet insbesondere eine ausreichende Durchlässigkeit in Kombination mit einer geeigneten Elastizität der Drahtwendel bei verschiedensten metallischen Drahtmaterialen. Zur Herstellung einer Öffnung kann die Drahtwendel an der gewünschten Stelle weichgezogen werden. Prinzipiell ist es aber auch möglich, zusätzlich oder anstelle einer Öffnung zwischen benachbarten Windungen der Drahtwendel beispielsweise eine Öffnung in der Führungsdrahtspitze vorzusehen. Dies kann z. B. eine in longitudinaler Richtung verlaufende Bohrung durch die Führungsdrahtspitze sein. Dadurch kann ein Fluid in distaler Richtung unmittelbar vor der Führungsdrahtspitze zu- und/oder abgeführt werden, was für spezielle Anforderungen vorteilhaft sein kann.

Der Abstand zwischen den benachbarten Windungen kann für spezielle Anwendungen und/oder bei speziellen Drahtmaterialien des Weiteren auch kleiner als 0.1 oder grösser als 0.5 Mal dem Drahtdurchmesser der Drahtwendel sein.

In einer weiteren bevorzugten Ausführungsform sind mehrere weitere benachbarte Windungen der Drahtwendel aneinander anliegend angeordnet, so dass wenigstens ein fluiddichter Abschnitt der Drahtwendel gebildet wird. In Kombination mit der wenigstens einen Öffnung ist es damit möglich, den Aus- und/oder Einlass für das zu- und/oder abzuführende Fluid an einer genau definierten Stelle der Drahtwendel vorzusehen. Dadurch kann die Zu- und/oder Abfuhr des Fluids im Wesentlichen punktuell an einer genau lokalisierten Stelle im Hohlorgan erfolgen. Damit ist es möglich, mit einer einzigen Drahtwendel, die z. B. aus einem einzigen Draht bestehen kann, sowohl ein Fluid zu leiten (ohne dass zusätzlich eine Membran als Mantel bzw. Schlauch vorgesehen sein muss) als es auch über einzelne Öffnungen aus bzw. einzubringen.

Es ist aber auch möglich, mehrere oder sämtliche Windungen der Drahtwendel beabstandet auszubilden, so dass das Fluid beispielsweise über den gesamten distalen Innenbereich der Drahtwendel oder gar über die gesamte Länge der Drahtwendel zu- und/oder abgeführt werden kann. Derartige Ausgestaltungen der Drahtwendel können insbesondere für eine grossflächige Applikation von Medikamenten vorteilhaft sein. Dabei ist natürlich zu beachten, dass ein längerer Abschnitt von beabstandeten Windungen nicht zu einem über die ganze Länge verteilten Abgabe bzw. Aufnahme des Fluids führt.

In einer besonders bevorzugten Ausführungsform, liegen in der Drahtwendel mehrere Öffnungen und mehrere fluiddichte Abschnitte vor, welche bevorzugt in einer alternierenden Abfolge und insbesondere in regelmässigen Abständen über eine gesamte Länge der Drahtwendel angeordnet sind. Die einzelnen Öffnungen sind dabei insbesondere im Wesentlichen von gleicher Grösse. Dadurch kann ein Fluid an mehreren beabstandeten Stellen gleichzeitig lokal zu- und/oder abgeführt werden. Dies ermöglicht insbesondere beim Zuführen eines Fluids eine präzise Dosierung.

Grundsätzlich können die Öffnungen aber auch in unregelmässigen Abständen vorliegen, sofern dies für spezielle Anwendungen zweckmässig erscheint.

Bevorzugt sind die fluiddichten Abschnitte der Drahtwendel röntgendicht ausgebildet. Hierfür besteht die Drahtwendel bevorzugt aus einem Platindraht, wobei ein Drahtdurchmesser insbesondere wenigstens 25 µm, bevorzugt 45 - 55 µm, beträgt. Dadurch sind die fluidddichten Abschnitte oder die Abschnitte der Drahtwendel mit aneinander liegenden Windungen in den üblicherweise eingesetzten bildgebenden Röntgenverfahren röntgendicht und können direkt visualisiert werden. Auf zusätzliche Röntgenmarker am Führungsdraht kann in diesem Fall verzichtet werden. Die Position der Drahtwendel lässt sich somit sehr genau bestimmen.

Bevorzugt sind die durch die beabstandete Windungen gebildeten Öffnungen der Drahtwendel röntgendurchlässig ausgebildet. Dies ist insbesondere durch einen Abstand zwischen den wenigstens zwei benachbarten Windungen von 0.1 - 0.5 Mal dem Drahtdurchmesser der Drahtwendel realisierbar. Damit ist eine direkte Visualisierung der Öffnungen in der Drahtwendel möglich, womit die Genauigkeit beim Zu- und/oder Abführen eines Fluids weiter verbessert wird. Bei mehreren regelmässig angeordneten röntgendurchlässigen Öffnungen mit dazwischen liegenden röntgendichten Abschnitten der Drahtwendel, kann die Lage des Führungsdrahts oder der Verlauf des Hohlorgans im dreidimensionalen Raum näher bestimmt werden.

Prinzipiell können die fluiddichten Abschnitte der Drahtwendel aber auch röntgendurchlässig und/oder die Öffnungen röntgendicht sein. Sofern erwünscht, ist es in diesen Fällen z. B. möglich, zusätzliche Röntgenmarker, z. B. Metallringe, am Führungsdraht anzubringen.

Weiter bevorzugt ist eine Elastizität der Drahtwendel grösser als eine Elastizität des Hohlschafts. Insbesondere ist ein Elastizitätsmodul der Drahtwendel geringer als ein Elastizitätsmodul des Hohlschafts. Dadurch ist ein distaler Abschnitt des Führungsdrahts elastischer als die in proximaler Richtung dahinter liegenden Bereiche des Führungsdrahts. Dadurch kann der elastischere distale Abschnitt des Führungsdraht besser durch die teilweise stark verzweigten und gebogenen Hohlorgane, z. B. Blutgefässe, gelenkt werden, während die hinteren Abschnitte eine ausreichende Steifigkeit zum Einschieben des Führungsdrahts aufweisen. Insgesamt werden dadurch die Einführbarkeit und die Positionierbarkeit des Führungsdrahts verbessert. Eine Feinabstimmung der Elastizitäten im distalen Bereich erfolgt, wie vorstehend ausgeführt, durch den Kerndraht.

Prinzipiell können die Elastizitäten von Kerndraht und Hohlschaft aber z. B. auch gleich sein, sofern dies für spezielle Anwendungen zweckmässig erscheint.

Besonders bevorzugt liegt der Hohlschaft in Form eines Stahlröhrchens vor, während die Drahtwendel insbesondere aus einem Platindraht besteht. Eine derartige Kombination hat sich im Hinblick auf eine gute Einführbarkeit in Kombination mit einer präzisen Positionierbarkeit als besonders geeignet erwiesen. Zudem sind die besagten sowohl Stahl als auch Platin gegenüber einer Vielzahl von in diesem Zusammenhang interessierenden Fluiden chemisch weitestgehend inert.

Grundsätzlich sind aber auch andere Materialen für den Hohlschaft und/oder die Drahtwendel einsetzbar. Insbesondere kann der Hohlschaft z. B. aus einem Kunststoffröhrchen bestehen.

Weiter vorteilhaft ist der Hohlschaft zwei- oder mehrteilig ausgebildet, wobei bevorzugt ein proximaler Abschnitt des Hohlschafts eine geringere Elastizität aufweist als ein distaler Abschnitt des Hohlschafts. Ein Elastizitätsmodul des proximalen Abschnitts ist dabei insbesondere grösser als ein Elastizitätsmodul des distalen Abschnitts des Hohlschafts. Weiter bevorzugt weist der distale Abschnitt des Hohlschafts eine geringere Flexibilität auf als die Drahtwendel. Insbesondere ist das Elastizitätsmodul des distalen Abschnitts des Hohlschafts grösser als das Elastizitätsmodul der Drahtwendel. Ein Aussendurchmesser des zwei- oder mehrteiligen Hohlschafts ist mit Vorteil im Wesentlichen über eine gesamte Länge des Hohlschafts konstant. Entsprechend ist auch ein Innendurchmesser des zwei- oder mehrteiligen Hohlschafts mit Vorteil im Wesentlichen über eine gesamte Länge des Hohlschafts konstant.

Derartige Führungsdrähte verfügen insbesondere über eine zur Führungsdrahtspitze zunehmende Elastizität. Durch den zwei- oder mehrteiligen Aufbau des Hohlschafts kann sich die Elastizitätszunahme in longitudinaler Richtung über einen längeren Bereich erstrecken, als bei einem einteiligen Hohlschaft. Damit lässt sich die Einführbarkeit und Positionierbarkeit des Führungsdrahts in einem Hohlorgan weiter verbessern.

Bei einem zwei- oder mehrteiligen Hohlschaft ist der Kerndraht bevorzugt im proximalen Abschnitt des Hohlschafts angebracht, wobei sich der Kerndraht insbesondere durch den distalen Abschnitt des Hohlschafts und die Drahtwendel bis zur Führungsdrahtspitze hin erstreckt. Dadurch lässt sich die Elastizitätszunahme des Führungsdrahts in longitudinaler Richtung besonders genau kontrollieren und Elastizitätssprünge an den Übergängen zwischen den verschiedenen Bereichen des Hohlschafts und der Drahtwendel können falls erforderlich ausgeglichen werden.

Grundsätzlich können aber auch einteilige Hohlschäfte verwendet werden. Auch möglich ist es, zwei- oder mehrteilige Hohlschäfte vorzusehen, welche in ihren distalen und proximalen Abschnitten beispielsweise über im Wesentlichen gleiche Elastizitäten verfügen. Falls erwünscht kann eine Elastizität in diesen Fällen z. B. über den Kerndraht gesteuert werden. Ebenso können die Aussen- und/oder Innendurchmesser des Hohlschafts in distaler Richtung beispielsweise abnehmend ausgebildet sein.

Speziell bevorzugt nimmt ein Durchmesser des Kerndrahts von einem proximalen Ende des Kerndrahts zu einem distalen Ende des Kerndrahts hin ab, bevorzugt so, dass eine Flexibilität des Führungsdrahts vom proximalen Ende des Kerndrahts zur Führungsdrahtspitze hin kontinuierlich abnimmt. Dies ist insbesondere unabhängig davon, ob ein ein-, zwei- oder mehrteiliger Hohlschaft vorliegt.

Falls zweckmässig kann der Kerndraht jedoch auch anders ausgebildet sein.

In einer bevorzugten Variante besteht der proximale Abschnitt aus einem Metallröhrchen, insbesondere einem Stahlröhrchen, und/oder der distale Abschnitt besteht aus einem Kunststoffröhrchen, insbesondere einem Polyimidröhrchen. Eine derartige Kombination hat sich im Hinblick auf eine gute Einführbarkeit in Kombination mit einer präzisen Positionierbarkeit besonders vorteilhaft erwiesen. Zudem sind die genannten Materialien gegenüber einer Vielzahl von in diesem Zusammenhang interessierenden Fluiden chemisch weitestgehend inert.

Für spezifische Anwendungen können aber auch andere Materialien oder Strukturen des Hohlschafts vorteilhaft sein.

Vorzugsweise haben die mehreren Teile den gleichen Aussendurchmesser. Das heisst, an den Übergängen gibt es keine Stufen. Gemäss dieser Ausführungsoption hat also der proximale Abschnitt (z. B. das Stahlröhrchen) und der distale Abschnitt (z. B. die einzelne Drahtwendel) und das diese beiden Abschnitte verbindende Zwischenröhrchen (Kunststoffröhrchen den gleichen Aussendurchmesser. Mit Vorteil ist auch der Innendurchmesser im Wesentlichen gleich gross. Zudem ist es bei den soeben beschriebenen Varianten jeweils besonders vorteilhaft, wenn der rohrartige Teil des Führungsdrahts ausschliesslich aus den drei Teilen mit dem gleichen Aussendurchmesser besteht und frei von einer zusätzlichen Membran (Ummantelung) ist.

Eine besonders bevorzugte Ausführungsform zeichnet sich also dadurch aus, dass die Drahtwendel aus einem einzigen Draht besteht, dass der Bereich der Drahtwendel frei von einer zusätzlichen Membran ist und dass genau drei verschiedene rohrartige Teile vorgesehen sind mit im Wesentlichen gleichem Aussendurchmesser.

Gemäss der Erfindung erstreckt sich ein den Kerndraht umgebender und aus einem distalen Ende des Hohlschafts herausragender und hohlzylindrischer rohrförmiger Stutzen teilweise in den Innenraum der Drahtwendel hinein. Ein distales Ende des rohrförmigen Stutzens liegt dabei erfindungsgemäss in proximaler Richtung vom distalen Ende der Drahtwendel beabstandet vor. Dadurch kann die Drahtwendel am Übergang zum Hohlschaft zusätzlich gestützt werden, was insbesondere die Stabilität der Verbindung erhöht und den Elastizitätssprung am Übergang reduziert. Aufgrund des von der Führungsdrahtspitze beabstandet angeordneten distalen Endes des rohrförmigen Stutzens ist eine Zu- und/oder Abfuhr über eine unmittelbar hinter der Führungsdrahtspitze in der Drahtwendel seitlich ausgebildete Öffnung weiterhin möglich. Erstreckt sich der rohrförmige Stutzen bis an die Führungsdrahtspitze, kann ein zu- und/oder abzuführendes Fluid beispielsweise über eine in longitudinaler Richtung in die Führungsdrahtspitze eingebrachte Öffnung erfolgen.

Ein derartiger Stutzen kann modifiziert werden.

Ein Aussendurchmesser des rohrförmigen Stutzens entspricht bevorzugt ungefähr einem Innendurchmesser der Drahtwendel. Dadurch wird eine optimale Abstützung der Drahtwendel erreicht. Weiter bevorzugt ist zudem ein Innendurchmesser des rohrförmigen Stutzens grösser als ein Aussendurchmesser des Kerndrahts im Bereich des rohrförmigen Stutzens. Damit bleibt neben dem Kerndraht ein Freiraum, welcher als Fluidkanal zur Durchleitung des zu- und/oder abzuführenden Fluids dient. Dies stellt eine besonders kompakte und herstellungstechnisch vorteilhafte Lösung dar.

Prinzipiell ist es aber auch denkbar, von den vorstehend genannten Durchmessern abzuweichen. Wird der Innendurchmesser des rohrförmigen Stutzens in etwa gleich dem Aussendurchmesser des Kerndrahts in diesem Bereich gewählt, so kann ein Fluidkanal wie vorstehend bereits ausgeführt beispielsweise im Kerndraht und/oder in einem separaten Fluidkanal neben dem rohrförmigen Stutzen realisiert werden.

Weiter bevorzugt ist der rohrförmige Stutzen innenseitig über eine fluiddurchlässige Verbindung mit dem Kerndraht und aussenseitig über eine weitere Verbindung mit der Drahtwendel verbunden. Dadurch wird insbesondere eine noch präzisere Bewegung des Führungsdrahts in einem Hohlorgan möglich, da Stauchungen und/oder Dehnungen der Drahtwendel in longitudinaler Richtung nahezu vollständig verhindert werden. Wie bereits vorstehend beschrieben, kann die fluiddurchlässige Verbindung eine asymmetrisch und/oder einseitig ausgebildete stoffschlüssige Verbindung, insbesondere eine Lotverbindung, zwischen rohrförmigen Stutzen und Kerndraht sein. Ebenso ist es möglich ein zusätzliches Röhrchen in die fluiddurchlässige Verbindung einzubetten. Dadurch bleibt im Bereich der fluiddurchlässigen Verbindung ein seitlich des Kerndrahts liegender kanalartiger Durchlass für das zu- und/oder abzuführende Fluid frei. Die weitere Verbindung zwischen rohrförmigem Stutzen und Drahtwendel kann z. B. als eine stoffschlüssige Verbindung, insbesondere eine Verschweissung, eine Verklebung und/oder eine Lotverbindung, vorliegen.

Auf derartige fluiddurchlässige Verbindungen und/oder weitere Verbindungen kann jedoch auch verzichtet werden.

Als besonders geeignet haben sich halbkugelförmige Aufsätze als Führungsdrahtspitzen herausgestellt. Die Führungsdrahtspitze des Führungsdrahts liegt daher erfindungsgemäss als halbkugelförmiger Aufsatz am distalen Ende der Drahtwendel vor. Derartige Aufsätze als Führungsdrahtspitzen sind im Besonderen atraumatisch. Die Führungsdrahtspitzen werden beispielsweise aus einem Kunststoffmaterial gefertigt.

Es sind aber auch anders ausgeformte Führungsdrahtspitzen möglich, sofern dies für spezielle Anwendungen erforderlich oder vorteilhaft ist.

Die erfindungsgemässen Führungsdrähte lassen sich insbesondere für diagnostische und/oder therapeutische Zwecke am menschlichen und/oder tierischen Körper einsetzen. Konkret können die erfindungsgemässen Führungsdrähte z. B. zur Behandlung der peripheren arteriellen Verschlusserkrankung oder der koronaren Herzerkrankung, zur Rekanalisation von Blutgefässen, zur flussverbessernden Behandlung von degenerierten Bypassgefässen, zur medikamentösen Therapie von Tumoren und/oder zur Druckmessung in einem Hohlorgan des menschlichen und/oder tierischen Körpers eingesetzt werden. Es sind aber auch andere Verwendungszwecke möglich.

Ein weiterer Aspekt der Offenbarung bezieht sich auf ein Verfahren zur diagnostischen und/oder therapeutischen Behandlung von Gefässen und/oder Tumoren. Dabei wird ein distaler Abschnitt eines erfindungsgemässen Führungsdrahts in ein Gefäss eingeführt, die Führungsdrahtspitze an einer zu behandelnden Stelle im Gefäss und/oder im Bereich eines Tumors positioniert, und anschliessend eine fluide Wirksubstanz durch das Lumen des Hohlschafts und die wenigstens eine Öffnung des Führungsdrahts in das zu behandelnde Gefäss und/oder den Tumor eingeleitet.

Die Abgabe der fluiden Wirksubstanz kann hierbei über Sekunden bis Stunden erfolgen und ist primär abhängig von der klinischen Indikationsstellung. Die Flussgeschwindigkeit und damit auch die abgegebene Substanzmenge ist im Wesentlichen abhängig vom applizierten Druck, der auf die fluide Wirksubstanz am proximalen Führungsdrahtende ausgeübt wird und den Substanzeigenschaften der applizierten fluiden Wirksubstanz selbst.

Die Einlage eines erfindungsgemässen Führungsdrahtes in ein Gefässe und/oder einen Tumor und die lokale und/oder punktuelle Applikation einer fluiden Wirksubstanz kann aufgrund der genauen Positionierbarkeit des erfindungsgemässen Führungsdrahts eine hohe und sehr genau absehbare Wirksamkeit zeigen.

Die fluide Wirksubstanz ist beispielsweise ein Kontrastmittel für ein bildgebendes Verfahren, eine thrombolytische Substanz, und/oder eine antithrombozytäre, embolisch, toxisch- und/oder antipoliferativ wirksame Substanz.

Vertreter von thrombolytischen Substanzen sind z. B. rt-PA, Metalyse, Streptokinase und/oder Urokinase. Ebenfalls geeignet als fluide Wirksubstanzen sind beispielsweise Kombinationen von antithrombozytären und antiplasmatisch wirksamen Substanzen. Hierbei können insbesondere Aspirin, Clopidogrel, Effient oder die Gruppe der GPIIb/IIIa Antagonisten eingesetzt werden. Als antipoliferativ wirksame Substanzen können beispielsweise Paclitaxel, Takrolimus, Sirolimus und/oder Everolimus eingesetzt werden. Je nach Behandlungsmethode sind aber auch andere Wirksubstanzen verwendbar.

Während der Rekanalisation von Gefässen im akuten Infarktstadium treten häufig sogenannte No-Reflow Phänomene bzw. mangelnde Rezirkulationen im Anschluss an eine Ischämie auf, die im Wesentlichen durch einen peripheren Thromboembolismus und/oder einen thrombotischen peripheren Gefässverschluss bedingt sind. Eine systemische thrombolytische Therapie ist teilweise nicht möglich, um die Thrombuslast zu lysieren, da es sich um alte Thromben handelt, die entweder von der thrombolytischen Substanz nicht erreicht oder, da alt, nicht lysiert werden können. Die Einlage eines erfindungsgemässen Führungsdrahts in die Gefässperipherie eines solchen Gefässes und die lokale Applikation von beispielsweise thrombolytischen Substanzen wie z. B. rt-PA, Metalyse, Streptokinase und/oder Urokinase kann hier aufgrund der unmittelbaren Nähe der wenigstens einen Öffnung des erfindungsgemässen Führungsdrahts zur thrombotischen Struktur thrombolytische Wirksamkeit entwickeln. Eine weitere Verbesserung des antegrade Flusses ist dabei auch zu erwarten durch eine Kombination von antithrombozytären und antiplasmatisch wirksamen Substanzen. Hierzu gehören insbesondere Aspirin, Clopidogrel, Effient oder die Gruppe der GPIIb/IIIa Antagonisten.

Degenerierte Bypassgefässe, die ebenfalls über einen langstreckigen Gefässprozess arteriosklerotisch verändert und thrombotisch belegt sind, neigen ebenfalls zu No-Reflow Phänomenen. Auch hier kann durch die Einlage eines erfindungsgemässen Führungsdrahts und die Abgabe von antiplasmatischer oder antithrombozytär wirksamen Substanzen eine mögliche Flussverbesserung erreicht werden.

Patienten mit diffusem Gefässbefall bei peripherer arterieller Verschlusserkrankung oder koronaren Herzerkrankung sind nur bedingt mit gängigen interventionellen Techniken therapierbar. Hier ist zu erwarten, dass bei der Einlage eines erfindungsgemässen Führungsdrahts und die Abgabe von antipoliferativ wirksamen Substanzen wie Paclitaxel, Takrolimus, Sirolimus und/oder Everolimus ein Einfluss auf die Lumenweite erreicht werden kann.

Spezielle Tumore, die klar definierte Blutversorgungen aufweisen, und nicht oder nur mit einem erhöhten operativen Risiko oder erheblicher Schädigung des sonstigen Gesundheitszustandes des Patienten entfernt werden können, können über die intravasale Einlage eines erfindungsgemässen Führungsdrahts medikamentös therapiert werden. In Frage kommende Substanzen sind hier embolischer-, toxischer- und antipoliferativer Natur.

Aus der nachfolgenden Detailbeschreibung und der Gesamtheit der Patentansprüche ergeben sich weitere vorteilhafte Ausführungsformen und Merkmalskombinationen der Erfindung.

### Kurze Beschreibung der Zeichnungen

Die zur Erläuterung des Ausführungsbeispiels verwendeten Zeichnungen zeigen:
- Fig. 1: Einen Längsschnitt durch einen ersten Führungsdraht mit einer unmittelbar hinter der Führungsdrahtspitze liegenden Durchlassöffnung in einer Drahtwendel und einer fluiddurchlässigen Verbindung zwischen Kerndraht und Drahtwendel;
- Fig. 2: Den Führungsdraht aus Fig. 1 im Querschnitt entlang der Linie A - A;
- Fig. 3: Den Führungsdraht aus Fig. 1 im Querschnitt entlang der Linie B - B;
- Fig. 4: Einen Längsschnitt durch einen zweiten Führungsdraht mit einer fluiddurchlässigen Verbindung in Form eines eingebetteten Röhrchens;
- Fig. 5: Den Führungsdraht aus Fig. 4 im Querschnitt entlang der Linie C - C;
- Fig. 6: Einen Längsschnitt durch einen dritten Führungsdraht mit mehreren beabstandeten Durchlassöffnungen in der Drahtwendel;
- Fig. 7: Einen Längsschnitt durch einen vierten, erfindungsgemässen Führungsdraht mit einem aus dem Hohlschaft in die Drahtwendel hinein ragenden rohrförmigen Stutzen;
- Fig. 8: Den Führungsdraht aus Fig. 7 im Querschnitt entlang der Linie D - D;
- Fig. 9: Einen Längsschnitt durch den in ein verengtes Blutgefäss eingeführten Führungsdraht aus Fig. 1.

Grundsätzlich sind in den Figuren gleiche Teile mit gleichen Bezugszeichen versehen.

### Wege zur Ausführung der Erfindung

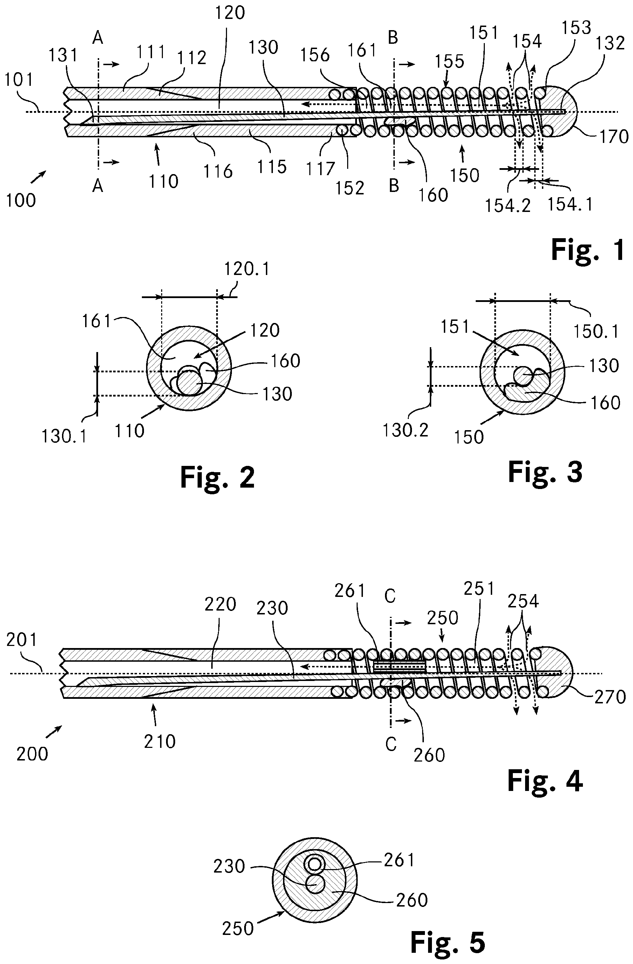

Die Fig. 1 - 3 zeigen einen ersten Führungsdraht 100, wobei Fig. 1 einen Längsschnitt entlang einer Längsmittelachse 101 des ersten Führungsdrahts 100 zeigt. Der Führungsdraht 100 umfasst einen zweiteiligen Hohlschaft 110, wobei ein proximaler Abschnitt 111 des Hohlschafts 110 beispielsweise aus einem hohlzylindrischen Stahlröhrchen gefertigt ist, während ein distaler Abschnitt 115 aus einem koaxial zum proximalen Abschnitt 111 angeordneten hohlzylindrischen Polyimidröhrchen besteht. Ein Querschnitt durch den proximalen Abschnitt 111 des Hohlschafts 110 ist in Fig. 2 gegeben. Aufgrund der Materialwahl ist die Elastizität des distalen Abschnitts 115 des Hohlschafts 110 dabei grösser als die Elastizität des proximalen Abschnitts 111.

Das distale Ende 112 des proximalen Abschnitts 111 verjüngt sich dabei in distaler Richtung bei konstantem Innendurchmesser, während sich das proximale Ende 116 des distalen Abschnitts 115 in proximaler Richtung komplementär zum distalen Ende 112 des proximalen Abschnitts 111 bei konstantem Aussendurchmesser konisch aufweitet. Die Innendurchmesser als auch die Aussendurchmesser der beiden Abschnitte 111, 115 sind im Wesentlichen gleich gross, so dass im Hohlschaft 110 ein durchgehendes kreiszylindrisches Lumen 120 mit einem konstanten Durchmesser 120.1 vorliegt.

Vom distalen Ende 117 des distalen Abschnitts 115 ragt eine koaxial angeordnete hohlzylindrische Drahtwendel 150 in distaler Richtung vom Hohlschafts 110 weg. Das proximale Ende 152 der Drahtwendel 150 ist dabei im distalen Ende 117 des distalen Abschnitts 115 durch Pressschweissung verankert. Die Drahtwendel besteht z. B. aus einem Platindraht mit einem Drahtdurchmesser von ca. 50 µm. Ein Querschnitt durch die Drahtwendel 150 ist in Fig. 3 wiedergegeben. Eine Flexibilität der Drahtwendel 150 ist dabei grösser als die Flexibilität des distalen Abschnitts 115 des Hohlschafts 110.

Am distalen Ende 153 der Drahtwendel ist eine Führungsdrahtspitze 170 in Form eines atraumatisch abgerundeten und halbkugelförmigen Aufsatzes aus Kunststoff angebracht. Die Führungsdrahtspitze 170 kann z. B. durch Pressschweissung mit der Drahtwendel 150 verbunden sein.

Die erste und die zweite, sowie die zweite und die dritte in proximaler Richtung unmittelbar hinter der Führungsdrahtspitze 170 liegenden Windungen der Drahtwendel 150 sind dabei beabstandet, so dass im Bereich der Führungsdrahtspitze eine fluiddurchlässige Durchlassöffnung 154 in der Drahtwendel 150 vorliegt. Ein erster Abstand 154.1 zwischen der ersten und der zweiten Windung, sowie ein zweiter Abstand 154.2 zwischen der zweiten und der dritten Windung entspricht dabei je ungefähr 0.3 Mal einem Drahtdurchmesser der Drahtwendel 150.

Die Durchlassöffnung 154 mündet direkt in den in proximaler Richtung an die Führungsdrahtspitze 170 angrenzenden distalen Innenbereich 151 der Drahtwendel 150. Mit anderen Worten verfügt der distale Innenbereich 151 der Drahtwendel 150 somit über eine nach aussen offene Durchlassöffnung 154 für ein ein- und/oder auszubringendes Fluid. Die übrigen Windungen der Drahtwendel 150 sind direkt aneinander anliegend angeordnet und bilden einen fluiddichten und röntgendichten Abschnitt 155 der Drahtwendel 150.

Ein Innendurchmesser 150.1 der Drahtwendel 150 ist über die gesamte Länge im Wesentlichen konstant und entspricht in etwa einem Innendurchmesser des Hohlschafts 110 bzw. einem Durchmesser des hohlzylindrischen Lumens 120.

Im proximalen Abschnitt 111 des Hohlschafts 110 ist des Weiteren das proximale Ende 131 eines Kerndrahts 130 an der Begrenzungsfläche des Lumens 120 bzw. im Innern des Hohlschafts 110 angeschweisst. Das proximale Ende 131 des Kerndrahts 130 ist dabei exzentrisch im Hohlschaft 110 angeordnet. Der Kerndraht 130 verläuft durch den distalen Abschnitt 115 und die Drahtwendel 130 hindurch bis in die Führungsdrahtspitze 170. Das distale Ende 132 des Führungsdrahts 130 ist mit der Führungsdrahtspitze 170, beispielsweise durch Pressschweissung, verbunden.

Der Kerndraht 130 besteht z. B. aus Stahl und weist einen von seinem proximalen Ende 131 zu seinem distalen Ende 132 hin abnehmenden Aussendurchmesser auf, wobei ein proximaler Aussendurchmesser 130.1 am proximalen Ende 131 des Kerndrahts 130 grösser ist als ein distaler Aussendurchmesser 130.2 des Kerndrahts im Bereich der Drahtwendel 150. Eine Flexibilität des Führungsdrahts 100 nimmt vom proximalen Ende 131 des Kerndrahts 130 zur Führungsdrahtspitze 170 hin im Wesentlichen kontinuierlich ab.

Der Aussendurchmesser 130.1, 130.2 des Kerndrahts 130 ist durchwegs kleiner als ein Durchmesser 120.1 des Lumens 120 und kleiner als ein Innendurchmesser 150.1 der Drahtwendel 150.

In einem proximalen Abschnitt der Drahtwendel 150 ist der Kerndraht 130 über eine fluiddurchlässige Verbindung 160 mit der Drahtwendel 150 verbunden. Die fluiddurchlässige Verbindung 160 ist beispielsweise eine stoffschlüssige Verbindung in Form einer Lotverbindung. Die fluiddurchlässige Verbindung 160 ist dabei bezüglich der Längsmittelachse 101 des Führungsdrahts 100 asymmetrisch oder einseitig ausgebildet, so dass im Bereich der fluiddurchlässigen Verbindung 160 neben dem Kerndraht 130 ein kanalartiger Durchlass 161 für das zu- und/oder abzuführende Fluid frei bleibt. Der distale Innenbereich 151 der Drahtwendel 150 kommuniziert somit über den kanalartigen Durchlass 161 mit einem proximalen Innenbereich 156 der Drahtwendel 150. Im Querschnitt bedeckt die fluiddurchlässige Verbindung ca. 40 % der Querschnittsfläche des Innenbereichs der Drahtwendel 150. Der Innendurchmesser 150.1 der Drahtwendel 150 ist im Bereich der fluiddurchlässigen Verbindung 160 ca. 3.5 Mal grösser als der proximale Aussendurchmesser 130.1 des Kerndrahts 130 in diesem Bereich.

Somit kommuniziert das Lumen 120 des ersten Führungsdrahts 100 über den neben dem Kerndraht 130 ausgebildeten kanalartigen Durchlass 161 mit dem distalen Innenbereich 151 der Drahtwendel 150, wobei der distale Innenbereich 151 wiederum über eine Durchlassöffnung 154 nach aussen zum Zu- und/oder Abführen eines Fluids verfügt.

Fig. 4 zeigt einen zweiten Führungsdraht 200 in einem Längsschnitt entlang seiner Längsmittellachse 201 während Fig. 5 einen Querschnitt im Bereich der fluiddurchlässigen Verbindung 260 des zweiten Führungsdrahts wiedergibt.

Der zweite Führungsdraht 200 verfügt ebenfalls über einen Hohlschaft 210 mit einem Lumen 220, wobei der Hohlschaft 210 und das Lumen 220 des zweiten Führungsdrahts 200 im Wesentlichen baugleich sind mit dem Hohlschaft 110 respektive dem Lumen 120 des ersten Führungsdrahts 100. Ebenso verfügt der zweite Führungsdraht 200 über eine am Hohlschaft 210 befestigte Drahtwendel 250 mit einer Führungsdrahtspitze 270 sowie einen Kerndraht 230. Auch die Drahtwendel 250, die Führungsdrahtspitze 270 sowie der Kerndraht 230 sind dabei im Wesentlichen baugleich mit den entsprechenden Teilen des ersten Führungsdrahts 100. Des Weiteren sind die genannten Teile des zweiten Führungsdrahts 200 in gleicher Weise zueinander angeordnet und untereinander befestigt wie die entsprechenden Teile des ersten Führungsdrahts 100. Entsprechend verfügt die Drahtwendel 250 des zweiten Führungsdrahts 200 auch über eine unmittelbar hinter der Führungsdrahtspitze 270 angeordnete Durchlassöffnung 254 für ein zu- und/oder abzuführendes Fluid.

Im Unterschied zum ersten Führungsdraht 100 liegt jedoch beim zweiten Führungsdraht 200 eine fluiddurchlässige Verbindung 260 als stoffschlüssige Verbindung mit einem darin eingebetteten und parallel zum Kerndraht verlaufenden Röhrchen 261 als kanalartiger Durchlass für das zu- und/oder abzuführende Fluid vor. Das Röhrchen 261 ist dabei z. B. rundum stoffschlüssig in eine Lotmasse eingebettet und mit der Drahtwendel 250 verbunden.

Somit kommuniziert das Lumen 220 des zweiten Führungsdrahts 200 über das neben dem Kerndraht 230 angeordnete Röhrchen 261, welches als kanalartiger Durchlass dient, mit dem distalen Innenbereich 251 der Drahtwendel 250, wobei der distale Innenbereich 251 wiederum über die Durchlassöffnung 254 zum Zu- und/oder Abführen eines Fluids verfügt.

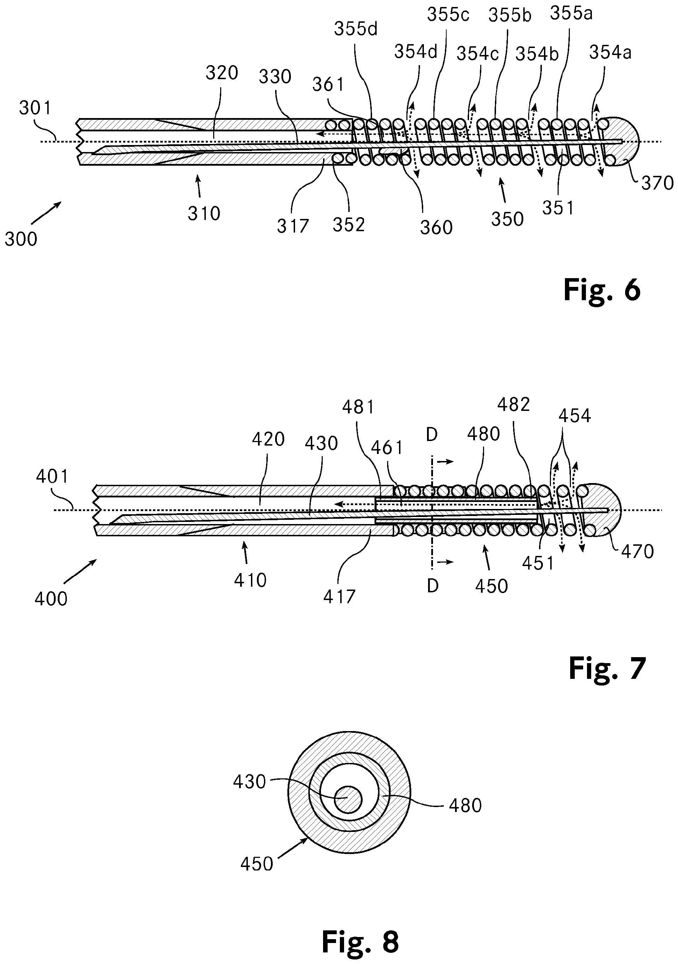

Fig. 6 zeigt einen dritten Führungsdraht 300 in einem Längsschnitt entlang seiner Längsmittelachse 301.

Der dritte Führungsdraht 300 verfügt ebenfalls über einen Hohlschaft 310 mit einem Lumen 320, wobei der Hohlschaft 310 und das Lumen 320 des dritten Führungsdrahts 300 im Wesentlichen baugleich sind mit dem Hohlschaft 110 respektive dem Lumen 120 des ersten Führungsdrahts 100. Ebenso verfügt der dritte Führungsdraht 300 über eine am Hohlschaft 310 befestigte Drahtwendel 350 mit einer Führungsdrahtspitze 370 sowie einen Kerndraht 330. Die Drahtwendel 350 besteht z.B. ebenfalls aus einem Platindraht mit einem Durchmesser von ca. 50 µm.

Im Unterschied zum ersten Führungsdraht 100 verfügt die Drahtwendel 350 jedoch über mehrere beabstandete Durchlassöffnungen 354a, 354b, 354c, 354d. Die erste und die zweite in proximaler Richtung unmittelbar hinter der Führungsdrahtspitze 370 liegenden Windungen der Drahtwendel 350 sind dabei voneinander beabstandet angeordnet, so dass im Bereich der Führungsdrahtspitze 370 eine erste fluiddurchlässige sowie röntgendurchlässige Durchlassöffnung 354a in der Drahtwendel 350 vorliegt.

In proximaler Richtung schliesst ein erster fluiddichter und röntgendichter Abschnitt 355a der Drahtwendel 350 an die erste fluiddurchlässige Durchlassöffnung 354a an. Der erste fluiddichte Abschnitt 355a wird dabei beispielsweise durch vier direkt aneinanderliegende Windungen der Drahtwendel 350 gebildet.

Auf den ersten fluiddichten Abschnitt 355a folgt in proximaler Richtung eine zweite fluiddurchlässige und röntgendurchlässige Durchlassöffnung 354b, welche durch zwei beabstandete Windungen der Drahtwendel 350 gebildet wird. Daran schliesst in proximaler Richtung ein zweiter fluiddichter und röntgendichter Abschnitt 355b der Drahtwendel 350 an. Wie bereits der erste fluiddichte Bereich 355a wird auch der zweite fluiddichte Bereich 355b z.B. durch vier direkt aneinander liegende Windungen der Drahtwendel 350 gebildet.

Auf den zweiten fluiddichten Abschnitt 355b folgt in proximaler Richtung eine dritte fluiddurchlässige und röntgendurchlässige Durchlassöffnung 354c, welche wiederum durch zwei beabstandete Windungen der Drahtwendel 350 gebildet ist. An die dritte Durchlassöffnung schliesst in proximaler Richtung ein dritter fluiddichter und röntgendichter Abschnitt 355c an, welcher ebenfalls durch vier direkt aneinander anliegende Windungen der Drahtwendel 350 gebildet wird.

Direkt auf den dritten fluiddichten Abschnitt 355c folgt in proximaler Richtung eine, durch zwei beabstandete Windungen der Drahtwendel 350 gebildete vierte, fluiddurchlässige und röntgendurchlässige Durchlassöffnung 354d.

An die vierte fluiddurchlässige Durchlassöffnung 354d grenzt in proximaler Richtung ein vierter aus vier direkt aneinander anliegenden Windungen der Drahtwendel gebildeter fluiddichter und röntgendichter Abschnitt 355d der Drahtwendel 350 an.

Die in proximaler Richtung letzten Drahtwindungen der Drahtwendel 350, welche das proximale Ende 352 der Drahtwendel bilden, sind im distalen Ende 317 des Hohlschafts 310 verschweisst.

Somit liegen entlang der gesamten Länge der Drahtwendel 350 mehrerer regelmässig beabstandete fluiddurchlässige und röntgendurchlässige Durchlassöffnungen 354a, 354b, 354c, 354d vor, welche durch mehrere fluiddichte und röntgendichte Abschnitte 355a, 355b, 355c, 355d separiert sind. Die beabstandeten Durchlassöffnungen 354a, 354b, 354c, 354d weisen dabei beispielsweise je eine Breite, gemessen in einer Richtung parallel zur Längsmittelachse 301, von ca. 0.5 Mal dem Drahtdurchmesser der Drahtwendel 350 auf.

Der aus dem Hohlschaft 310 heraus, durch die Drahtwendel 350 hindurch bis in der Führungsdrahtspitze ragende Kerndraht 330 ist im Bereich des vierten fluiddichten Abschnitts 355d mit der Drahtwendel 350 über eine fluiddurchlässige Verbindung 360 verbunden. Die fluiddurchlässige Verbindung 360 ist im Wesentlichen gleich ausgebildet wie die fluiddurchlässige Verbindung 160 des ersten Führungsdrahts 100 und liegt beispielsweise als stoffschlüssige Verbindung in Form einer Lotverbindung vor. Die fluiddurchlässige Verbindung 360 ist entsprechend bezüglich der Längsmittelachse 301 des dritten Führungsdrahts 300 asymmetrisch oder einseitig ausgebildet, so dass im Bereich der fluiddurchlässigen Verbindung 360 neben dem Kerndraht 130 ein kanalartiger Durchlass 361 für das zu- und/oder abzuführende Fluid frei bleibt. Im Querschnitt bedeckt die fluiddurchlässige Verbindung ca. 40 % der Querschnittsfläche des Innenbereichs der Drahtwendel 350. Der Innendurchmesser der Drahtwendel 350 ist im Bereich der fluiddurchlässigen Verbindung 360 ca. 3.5 Mal grösser als der Aussendurchmesser des Kerndrahts 330 in diesem Bereich.

Somit kommuniziert das Lumen 320 des dritten Führungsdrahts 300 über den neben dem Kerndraht 330 ausgebildeten kanalartigen Durchlass 361 mit dem distalen Innenbereich 351 der Drahtwendel 350, wobei der distale Innenbereich 351 wiederum über die vier Durchlassöffnungen 354a, 354b, 354c, 354d zum Zu- und/oder Abführen eines Fluids verfügt.

Die Fig. 7 und 8 zeigen einen vierten, erfindungsgemässen Führungsdraht 400 in einem Längsschnitt entlang der Längsachse 401 sowie in einem Querschnitt. Der Hohlschaft 410, die Drahtwendel 450, die Führungsdrahtspitze 470 sowie der Kerndraht 430 des vierten Führungsdrahts 400 sind im Wesentlichen baugleich mit den entsprechenden Teilen des ersten Führungsdrahts 100. Entsprechend verfügt die Drahtwendel 450 über eine in proximaler Richtung unmittelbar hinter der Führungsdrahtspitze 470 liegende fluiddurchlässige Durchlassöffnung 454, welche durch drei beabstandete Windungen der Drahtwendel gebildet wird und mit dem distalen Innenbereich 451 der Drahtwendel kommuniziert. Die Durchlassöffnung 454 der vierten Drahtwendel 450 entspricht im Wesentlichen der Durchlassöffnung 154 des ersten Führungsdrahts 100.

Im Unterschied zum ersten Führungsdraht 100 verfügt der vierte Führungsdraht 400 jedoch zusätzlich über einen rohrförmigen Stutzen 480 in Form eines hohlzylindrischen Röhrchens, welches koaxial zur Längsmittelachse 401 des vierten Führungsdrahts 400 aus dem Hohlschafts 410 in den Innenbereich der Drahtwendel 450 hineinragt. Ein proximales Ende 481 des rohrförmigen Stutzens 480 ist dabei in einem distalen Ende 417 des Hohlschafts 410 verankert, z. B. durch Verklebung und/oder Verschweissung. Der Aussendurchmesser des rohrförmigen Stutzens 480 entspricht dabei im Wesentlichen dem Innendurchmesser des Hohlschafts 410 und dem Innendurchmesser der Drahtwendel 450.

Ein proximaler Abschnitt des rohrförmigen Stutzens 480 ist zum Beispiel über eine Klebverbindung mit dem proximalen Abschnitt der Drahtwendel 450 verbunden.

Ein distales Ende 482 des rohrförmigen Stutzens 480 ist in distaler Richtung unmittelbar vor der Durchlassöffnung 454 angeordnet, so dass die Durchlassöffnung 454 nicht vom rohrförmigen Stutzen 480 verdeckt und somit frei bleibt.

Der Kerndraht 430 des vierten Führungsdrahts 400 verläuft durch den rohrförmigen Stutzen 480 hindurch, wobei ein Innendurchmesser des rohrförmigen Stutzens 480 durchwegs kleiner ist als ein Aussendurchmesser des Kerndrahts 430 in diesem Bereich. Damit bleibt im rohrförmigen Stutzen 480 neben dem Kerndraht 430 ein kanalartiger Durchlass 461 zum Durchleiten eines zu- und/oder abzuführenden Fluids frei. Das Lumen 420 des vierten Führungsdrahts 400 kommuniziert somit über den im rohrförmigen Stutzen 480 vorliegenden kanalartigen Durchlass 461 mit dem distalen Innenbereich 451 der Drahtwendel 450, welcher wiederum über eine fluiddurchlässige Durchlassöffnung 454 zum Zu- und/oder Abführen eines Fluids verfügt.

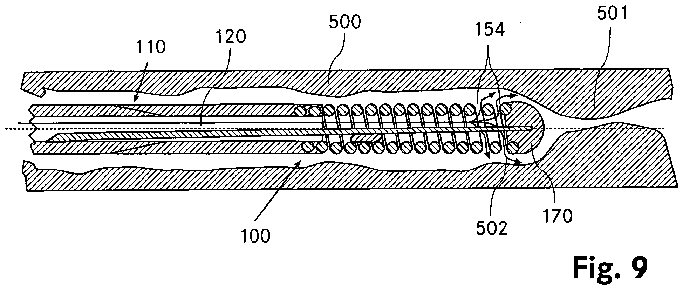

Fig. 9 zeigt den ersten Führungsdraht 100 aus den Fig. 1 - 3 nach dem Einschieben in ein Blutgefäss 500. Die Führungsdrahtspitze 170 liegt dabei unmittelbar vor einer verengten Stelle 501 im Blutgefäss 500, welche z.B. medikamentös behandelt werden soll. Im Bereich des ausserhalb des Körpers liegenden proximalen Endes des Führungsdrahts 100 (in Fig. 9 nicht dargestellt) kann das zu applizierende Medikament 502 in das Lumen 120 eingeleitet und über die unmittelbar hinter der Führungsdrahtspitze 170 liegende Durchlassöffnung 154 in das Gefäss 500 abgegeben werden. Da die Durchlassöffnung 154 unmittelbar im Bereich der Führungsdrahtspitze 170 liegt, gelangt das zu applizierende Medikament relativ präzise in den Bereich der zu behandelnden Verengung 501.

Die vorstehend beschriebenen Ausführungsformen sind lediglich als illustrative Beispiele zu verstehen, welche im Rahmen der Erfindung beliebig abgewandelt werden können.

So ist es bei sämtlichen Führungsdrähten 100, 200, 300, 400 beispielsweise möglich, anstelle der zweiteiligen Hohlschäfte 110, 210, 310, 410 einteilige Hohlschäfte vorzusehen. Diese können z. B. in Form eines Stahlröhrchens vorliegen. Die Drahtwendel kann in diesem Fall beispielsweise stirnseitig an den Hohlschaft angeschweisst werden. Auch möglich sind natürlich mehrteilige Hohlschäfte mit drei, vier oder noch separaten Teilabschnitten. Die Hohlschäfte 110, 210, 310, 410, unabhängig davon ob ein-, zwei- oder mehrteilig, können in distaler Richtung z. B. auch verjüngend ausgebildet sein, um insbesondere die Einführbarkeit zu verbessern.

Der distale Endbereich 112 des proximalen Abschnitts 111 und/oder der proximale Endbereich 116 des distalen Abschnitts 115 des Hohlschafts 110 können auch anders ausgeformt und/oder verbunden sein. Die beiden Abschnitte 111, 115 können z. B. über je eine senkrecht zur Längsmittelachse 101 vorliegende ringförmige Stirnfläche auf Stoss zusammengefügt und miteinander verklebt und/oder verschweisst sein.

Ebenso können die Führungsdrahtspitzen 170, 270, 370, 470 auch anders ausgeformt und z. B. an einen speziellen Anwendungszweck angepasst sein.

Zusätzlich zu den fluiddurchlässigen Verbindungen 160, 260, 360 können auch noch weitere Verbindungen zwischen Kerndraht 130, 230, 330 und Drahtwendel 150, 250, 350 ausgebildet sein. Es ist prinzipiell aber auch möglich, ersatzlos auf die fluiddurchlässigen Verbindungen 160, 260, 360 zu verzichten. Ebenso kann beim vierten Führungsdraht 400 eine zusätzliche fluiddurchlässige Verbindung zwischen Kerndraht und rohrförmigem Stutzen 480 ausgebildet werden, um z. B. die Stabilität der Drahtwendel zusätzlich zu erhöhen.

Bei der fluiddurchlässigen Verbindung 260 des zweiten Führungsdrahts 200 können auch zusätzlich zum Röhrchen 261 weitere Röhrchen vorgesehen werden, beispielsweise um die fluiddurchlässige Querschnittsfläche zu erhöhen.

Auch ist es grundsätzlich möglich, die Drahtwendel 150, 250, 350, 450 in distaler Richtung zu den Führungsdrahtspitzen hin verjüngend auszubilden, sofern dies zweckdienlich erscheint. Dies kann unter Umständen die Einführbarkeit der Führungsdrähte verbessern.

Beim dritten Führungsdraht 300 können auch mehr oder weniger als die vier Durchlassöffnungen 354a, 354b, 354c, 354d der Drahtwendel 350 vorgesehen werden. So ist es beispielsweise denkbar, sämtliche Windungen der Drahtwendel 350 beabstandet auszubilden, so dass ein Fluid über die gesamte Länge der Drahtwendel zu- und/oder abgeführt werden kann. Eine entsprechende Ausbildung ist auch bei den Drahtwendeln 150, 250 der ersten beiden Führungsdrähte 100, 200 möglich.

Beim ersten Führungsdraht 100 kann das proximale Ende 131 des Kerndrahts 130 grundsätzlich auch im distalen Abschnitt 115 des Hohlschafts 110 angebracht sein. Der Kerndraht 130 kann auch Bereiche mit stufenartig abnehmenden und/oder zunehmenden Aussendurchmessern aufweisen, um z. B. starke Elastizitätssprünge auszugleichen. Prinzipiell kann der Kerndraht aber auch einen entlang der gesamten Länge konstanten Aussendurchmesser aufweisen.

## Patentansprüche

1. Führungsdraht (100) für einen Katheter, welcher zum Einbringen und/oder Entnehmen von Fluiden in menschlichen und/oder tierischen Hohlorganen, insbesondere in Blutgefässen, ausgelegt ist, umfassend
a) einen länglichen Hohlschaft (110) mit einem Lumen (120) zum Zu- und/oder Abführen des Fluids,
b) sowie eine koaxial an einem distalen Ende (117) des Hohlschafts (110) anschliessende Einführhilfe in Form einer flexiblen Drahtwendel (150) mit einer an einem distalen Ende (153) der Drahtwendel angeordneten Führungsdrahtspitze (170), wobei die Führungsdrahtspitze als halbkugelförmiger Aufsatz am distalen Ende der Drahtwendel vorliegt,
c) wobei zur Steuerung einer Flexibilität des Führungsdrahts (100) im Lumen (120) des Hohlschafts (110) ein Kerndraht (130) angeordnet ist, welcher sich aus dem Lumen (120) heraus in einer longitudinalen Richtung durch die Drahtwendel (150) hindurch bis zur Führungsdrahtspitze (170) hin erstreckt,
d) wobei ein in einer proximalen Richtung an die Führungsdrahtspitze (170) angrenzender distaler Innenbereich (151) der Drahtwendel (150) über einen neben dem Kerndraht (130) ausgebildeten Fluidkanal (151, 161, 155) mit dem Lumen (120) des Hohlschafts (110) kommuniziert
e) und über wenigstens eine nach aussen offene Durchlassöffnung (154) für das ein- und/oder auszubringende Fluid verfügt,
f) wobei zur Erzeugung der wenigstens einen Durchlassöffnung (154) wenigstens zwei benachbarte Windungen der Drahtwendel (150) voneinander beabstandet angeordnet sind,
und **dadurch gekennzeichnet, dass**
g) sich ein den Kerndraht (430) umgebender und aus einem distalen Ende (417) des Hohlschafts (410) herausragender und hohlzylindrischer rohrförmiger Stutzen (480) teilweise in den Innenraum der Drahtwendel (450) hinein erstreckt, so dass ein distales Ende (482) des rohrförmigen Stutzens (480) vom distalen Ende der Drahtwendel (450) in proximaler Richtung beabstandet vorliegt.

2. Führungsdraht nach Anspruch 1, **dadurch gekennzeichnet, dass** in einem Bereich eines proximalen Endes (152) der Drahtwendel (150) eine fluiddurchlässige Verbindung (160) zwischen Drahtwendel (150) und Kerndraht (130) vorliegt, so dass die Drahtwendel (150) gegenüber dem Kerndraht (130) mechanisch fixiert ist und der distale Innenbereich (151) der Drahtwendel (150) mit einem proximal der fluiddurchlässigen Verbindung (160) vorliegenden proximalen Innenbereich (156) der Drahtwendel (150) kommuniziert.

3. Führungsdraht nach Anspruch 2, **dadurch gekennzeichnet, dass** die fluiddurchlässige Verbindung (160) als eine bezüglich einer Längsmittelachse (101) der Drahtwendel (150) asymmetrisch und/oder einseitig ausgebildete stoffschlüssige Verbindung, insbesondere eine Lotverbindung, zwischen Drahtwendel (150) und Kerndraht (130) vorliegt, so dass im Bereich der fluiddurchlässigen Verbindung (160) ein seitlich des Kerndrahts (130) liegender kanalartiger Durchlass (161) für das zu- und/oder abzuführende Fluid frei bleibt.

4. Führungsdraht nach Anspruch 2, **dadurch gekennzeichnet, dass** die fluiddurchlässige Verbindung (260) als stoffschlüssige Verbindung, insbesondere als Lotverbindung, ausgebildet ist, wobei ein parallel zum Kerndraht (230) verlaufendes und in die stoffschlüssige Verbindung (260) eingebettetes Röhrchen (261) als kanalartiger Durchlass für das zu- und/oder abzuführende Fluid vorliegt.

5. Führungsdraht nach einem der Ansprüche 1 - 4, **dadurch gekennzeichnet, dass** der Kerndraht (130) in einer proximalen Richtung vom proximalen Ende (152) der Drahtwendel (150) beabstandet im Lumen (120) fixiert ist und insbesondere exzentrisch bezüglich einer longitudinalen Achse des Lumens an einer Begrenzungsfläche des Lumens (120) anliegend angeordnet ist.

6. Führungsdraht nach einem der Ansprüche 1 - 5, **dadurch gekennzeichnet, dass** ein Aussendurchmesser (130.1, 130.2) des Kerndrahts (130) geringer ist als ein Innendurchmesser (120.1) des Lumens (120) und/oder geringer als ein Innendurchmesser (150.1) der Drahtwendel (150), wobei bevorzugt der Innendurchmesser (120.1) des Lumens (120) und/oder der Innendurchmesser (150.1) der Drahtwendel (150) 3 - 4 Mal grösser ist, als der Aussendurchmesser (130.2) des Kerndrahts (130) im Bereich der Drahtwendel (150).

7. Führungsdraht nach einem der Ansprüche 1 - 6, **dadurch gekennzeichnet, dass** zwischen den wenigstens zwei benachbarten Windungen ein Abstand (154.1, 154.1) vorliegt, welcher 0.1 - 0.5 Mal einem Drahtdurchmesser der Drahtwendel (150) entspricht.

8. Führungsdraht nach einem der Ansprüche 1 - 7, **dadurch gekennzeichnet, dass** ein Innendurchmesser (150.1) der Drahtwendel (150) über die gesamte Länge im Wesentlichen konstant ist und in etwa einem Innendurchmesser des Hohlschafts 110 bzw. einem Durchmesser des hohlzylindrischen Lumens 120 entspricht.

9. Führungsdraht nach Anspruch 8, **dadurch gekennzeichnet, dass** mehrere Durchlassöffnungen (354a, 354b, 354c, 354d) und mehrere fluiddichte Abschnitte (355a, 355b, 355c, 355d) vorliegen, welche bevorzugt in einer alternierenden Abfolge und insbesondere in regelmässigen Abständen über eine gesamte Länge der Drahtwendel (350) angeordnet sind.

10. Führungsdraht nach einem der Ansprüche 1 - 9, **dadurch gekennzeichnet, dass** eine Elastizität der Drahtwendel (150) grösser ist als eine Elastizität des Hohlschafts (110).

11. Führungsdraht nach einem der Ansprüche 1 - 10, **dadurch gekennzeichnet, dass** der Hohlschaft (110) in Form eines Stahlröhrchens vorliegt und die Drahtwendel (150) insbesondere aus einem Platindraht besteht.

12. Führungsdraht nach einem der Ansprüche 1 - 11, **dadurch gekennzeichnet, dass** der Hohlschaft (110) zwei- oder mehrteilig ausgebildet ist, wobei ein proximaler Abschnitt (111) des Hohlschafts (110) eine geringere Elastizität aufweist als ein distaler Abschnitt (115) des Hohlschafts (110).

13. Führungsdraht nach Anspruch 12, **dadurch gekennzeichnet, dass** der proximale Abschnitt (111) aus einem Metallröhrchen, insbesondere einem Stahlröhrchen, und der distale Abschnitt (115) aus einem Kunststoffröhrchen, insbesondere einem Polyimidröhrchen, besteht.

14. Führungsdraht nach einem der Ansprüche 12 - 13, **dadurch gekennzeichnet, dass** der distale Abschnitt (115) des Hohlschafts eine geringere Flexibilität aufweist als die Drahtwendel (150).

15. Führungsdraht nach einem der Ansprüche 1 bis 14, **dadurch gekennzeichnet, dass** ein Aussendurchmesser des rohrförmigen Stutzens (480) ungefähr einem Innendurchmesser der Drahtwendel (450) entspricht und bevorzugt ein Innendurchmesser des rohrförmigen Stutzens (480) grösser ist als ein Aussendurchmesser des Kerndrahts (430) im Bereich des rohrförmigen Stutzens (480).

16. Führungsdraht nach einem der Ansprüche 1 - 15, **dadurch gekennzeichnet, dass** ein Durchmesser des Kerndrahts (130) von einem proximalen Ende (131) zu einem distalen Ende (132) hin abnimmt, bevorzugt so, dass eine Flexibilität des Führungsdrahts (130) vom proximalen Ende (131) des Kerndrahts (130) zur Führungsdrahtspitze (170) hin kontinuierlich abnimmt.

17. Führungsdraht nach einem der Ansprüche 1 - 16, **dadurch gekennzeichnet, dass** die Führungsdrahtspitze (170) aus einem Kunststoffmaterial gefertigt ist.

18. Führungsdraht nach einem der Ansprüche 1 - 17, **dadurch gekennzeichnet, dass** die Drahtwendel aus einem einzige Draht besteht, dass der Bereich der Drahtwendel frei von einer zusätzlichen Membran ist.

19. Führungsdraht nach einem der Ansprüche 12 - 18, **dadurch gekennzeichnet, dass** genau drei verschiedene rohrartige Teile vorgesehen sind mit im Wesentlichen gleichem Aussendurchmesser, nämlich als erster rohrartiger Teil der proximale Abschnitt (111) des Hohlschafts (110), als zweiter rohrartiger Teil die Drahtwendel (150) und als dritter rohrartiger Teil ein die ersten beiden Teile verbindendes Zwischenröhrchen.

20. Führungsdraht nach einem der Ansprüche 1 - 19, **dadurch gekennzeichnet, dass** das proximale Ende der Drahtwendel (150) im distalen Ende (117) des distalen Abschnitts (115) des Hohlschafts verankert ist.

## Claims

1. Guidewire (100) for a catheter, designed to introduce and/or remove fluids in human and/or animal hollow organs, in particular in blood vessels, comprising
a) an elongate hollow shaft (110) with a lumen (120) for delivering and/or withdrawing the fluid,
b) and an insertion aid in the form of a flexible wire coil (150) attached coaxially to a distal end (117) of the hollow shaft (110), with a guidewire tip (170) arranged at a distal end (153) of the wire coil, wherein the guidewire tip is present as a hemispherical attachment on the distal end of the wire coil,
c) wherein a core wire (130) is arranged in the lumen (120) of the hollow shaft (110) in order to control a flexibility of the guidewire (100), which core wire (130) extends out of the lumen (120) in a longitudinal direction right through the wire coil (150) to the guidewire tip (170),
d) wherein a distal inner region (151) of the wire coil (150), adjacent to the guidewire tip (170) in a proximal direction, communicates with the lumen (120) of the hollow shaft (110) via a fluid channel (151, 161, 155) formed alongside the core wire (130)
e) and has at least one outwardly open through-opening (154) for the fluid that is to be introduced and/or removed,
f) wherein at least two adjacent windings of the wire coil (150) are arranged spaced apart from each other in order to generate the at least one through-opening (154),
and **characterized in that**
g) a hollow cylindrical tubular stub (480), surrounding the core wire (430) and protruding from a distal end (417) of the hollow shaft (410), extends partially into the interior of the wire coil (450), such that a distal end (482) of the tubular stub (480) is spaced apart in the proximal direction from the distal end of the wire coil (450).

2. Guidewire according to Claim 1, **characterized in that** a fluid-permeable connection (160) between wire coil (150) and core wire (130) is present in a region of a proximal end (152) of the wire coil (150), such that the wire coil (150) is mechanically fixed relative to the core wire (130), and the distal inner region (151) of the wire coil (150) communicates with a proximal inner region (156) of the wire coil (150) lying proximally from the fluid-permeable connection (160).

3. Guidewire according to Claim 2, **characterized in that** the fluid-permeable connection (160) is present as an integrally bonded connection which is designed asymmetrically and/or to one side with respect to a longitudinal centre axis (101) of the wire coil (150), in particular a soldered connection, between wire coil (150) and core wire (130), such that, in the region of the fluid-permeable connection (160), a channel-like passage (161) lying to the side of the core wire (130) remains free for the fluid that is to be delivered and/or withdrawn.

4. Guidewire according to Claim 2, **characterized in that** the fluid-permeable connection (260) is designed as an integrally bonded connection, in particular as a soldered connection, wherein a tube (261) extending parallel to the core wire (230) and embedded in the integrally bonded connection (260) is present as a channel-like passage for the fluid that is to be delivered and/or withdrawn.

5. Guidewire according to one of Claims 1-4, **characterized in that** the core wire (130) is fixed in the lumen (120) at a distance in a proximal direction from the proximal end (152) of the wire coil (150) and, in particular, is arranged bearing on a boundary surface of the lumen (120), eccentrically with respect to a longitudinal axis of the lumen.

6. Guidewire according to one of Claims 1-5, **characterized in that** an external diameter (130.1, 130.2) of the core wire (130) is smaller than an internal diameter (120.1) of the lumen (120) and/or smaller than an internal diameter (150.1) of the wire coil (150), wherein the internal diameter (120.1) of the lumen (120) and/or the internal diameter (150.1) of the wire coil (150) is preferably 3-4 times greater than the external diameter (130.2) of the core wire (130) in the region of the wire coil (150).

7. Guidewire according to one of Claims 1-6, **characterized in that**, between the at least two adjacent windings, there is a spacing (154.1, 154.1) that corresponds to 0.1-0.5 times a wire diameter of the wire coil (150).

8. Guidewire according to one of Claims 1-7, **characterized in that** an internal diameter (150.1) of the wire coil (150) is substantially constant along the entire length and corresponds approximately to an internal diameter of the hollow shaft (110) or to a diameter of the hollow cylindrical lumen (120).

9. Guidewire according to Claim 8, **characterized in that** several through-openings (354a, 354b, 354c, 354d) and several fluid-tight portions (355a, 355b, 355c, 355d) are present, which are preferably arranged in an alternating sequence and in particular at regular intervals along an entire length of the wire coil (350).

10. Guidewire according to one of Claims 1-9, **characterized in that** an elasticity of the wire coil (150) is greater than an elasticity of the hollow shaft (110).

11. Guidewire according to one of Claims 1-10, **characterized in that** the hollow shaft (110) is present in the form of a steel tube, and the wire coil (150) is composed in particular of a platinum wire.

12. Guidewire according to one of Claims 1-11, **characterized in that** the hollow shaft (110) is designed in two or more parts, wherein a proximal portion (111) of the hollow shaft (110) has less elasticity than a distal portion (115) of the hollow shaft (110).

13. Guidewire according to Claim 12, **characterized in that** the proximal portion (111) is composed of a metal tube, in particular a steel tube, and the distal portion (115) is composed of a plastic tube, in particular a polyimide tube.

14. Guidewire according to either of Claims 12 and 13, **characterized in that** the distal portion (115) of the hollow shaft has less flexibility than the wire coil (150).

15. Guidewire according to one of Claims 1 to 14, **characterized in that** an external diameter of the tubular stub (480) corresponds approximately to an internal diameter of the wire coil (450), and an internal diameter of the tubular stub (480) is preferably larger than an external diameter of the core wire (430) in the region of the tubular stub (480) .

16. Guidewire according to one of Claims 1-15, **characterized in that** a diameter of the core wire (130) decreases from a proximal end (131) towards a distal end (132), preferably such that a flexibility of the guidewire (130) decreases continuously from the proximal end (131) of the core wire (130) towards the guidewire tip (170).

17. Guidewire according to one of Claims 1-16, **characterized in that** the guidewire tip (170) is produced from a plastic material.

18. Guidewire according to one of Claims 1-17, **characterized in that** the wire coil is composed of a single wire, **in that** the region of the wire coil is free of an additional membrane.

19. Guidewire according to one of Claims 12-18, **characterized in that** that precisely three different tubular parts are provided with substantially the same external diameter, namely, as first tubular part the proximal portion (111) of the hollow shaft (110), as second tubular part the wire coil (150), and as third tubular part an intermediate tube connecting the first two parts.

20. Guidewire according to one of Claims 1-19, **characterized in that** the proximal end of the wire coil (150) is anchored in the distal end (117) of the distal portion (115) of the hollow shaft.

## Revendications

1. Fil de guidage (100) destiné à un cathéter conçu pour introduire et/ou éliminer des fluides dans des organes creux humains et/ou animaux, en particulier dans des vaisseaux sanguins, ledit fil de guidage comprenant
a) une tige creuse allongée (110) pourvue d'une lumière (120) pour alimenter et/ou évacuer le fluide,
b) et un auxiliaire d'insertion, se présentant sous la forme d'un fil hélicoïdal flexible (150), qui fait suite coaxialement à une extrémité distale (117) de la tige creuse (110) et qui comporte une pointe de fil de guidage (170) disposée sur une extrémité distale (153) du fil hélicoïdal, la pointe de fil de guidage étant présente à l'extrémité distale du fil hélicoïdal sous la forme d'un chapeau hémisphérique,
c) un fil central (130), qui s'étend hors de la lumière (120) dans une direction longitudinale à travers le fil hélicoïdal (150) jusqu'à la pointe de fil de guidage (170), étant disposé dans la lumière (120) de la tige creuse (110) pour commander la flexibilité du fil de guidage (100),
d) une région intérieure distale (151) du fil hélicoïdal (150), adjacente à la pointe de fil de guidage (170) dans une direction proximale, communiquant avec la lumière (120) de la tige creuse (110) par le biais d'un conduit de fluide (151, 161, 155) formé à côté du fil central (130),
e) et disposant d'au moins une ouverture de passage (154) ouverte vers l'extérieur et destinée à l'introduction et/ou à l'évacuation du fluide,
f) au moins deux enroulements adjacents du fil hélicoïdal (150) étant disposés à distance l'un de l'autre pour produire l'au moins une ouverture de passage (154),
et **caractérisé en ce que**
g) un ajutage tubulaire (480) en forme de cylindre creux entourant le fil central (430) et saillant d'une extrémité distale (417) de la tige creuse (410) s'étendant partiellement dans l'espace intérieur du fil hélicoïdal (450) de sorte qu'une extrémité distale (482) de l'ajutage tubulaire (480) soit espacée de l'extrémité distale du fil hélicoïdal (450) dans la direction proximale.

2. Fil de guidage selon la revendication 1, **caractérisé en ce que**, dans une région d'une extrémité proximale (152) du fil hélicoïdal (150), une liaison (160) perméable aux fluides est présente entre le fil hélicoïdal (150) et le fil central (130) de sorte que le fil hélicoïdal (150) est fixée mécaniquement par rapport au fil central (130) et la région intérieure distale (151) du fil hélicoïdal (150) communique avec une région intérieure proximale (156) du fil hélicoïdal (150) qui est présente du côté proximal de la liaison (160) perméable aux fluides.

3. Fil de guidage selon la revendication 2, **caractérisé en ce que** la liaison (160) perméable aux fluides est présente sous la forme d'une liaison de matière asymétrique par rapport à un axe central longitudinal (101) du fil hélicoïdal (150) et/ou formée d'un seul côté, en particulier une liaison par brasage, entre le fil hélicoïdal (150) et le fil central (130) de sorte qu'un passage (161) en forme de conduit, situé latéralement par rapport au fil central (130) et destiné à l'amenée et/ou l'évacuation de fluide, reste libre dans la région de la liaison (160) perméable aux fluides.

4. Fil de guidage selon la revendication 2, **caractérisé en ce que** la liaison (260) perméable aux fluides est conçue comme une liaison de matière, en particulier une liaison par brasage, un tube (261) parallèle au fil central (230) et intégré dans la liaison de matière (260) étant présent sous la forme d'un passage en forme de conduit destiné à l'amenée et/ou l'évacuation de fluide.

5. Fil de guidage selon l'une des revendications 1 à 4, **caractérisé en ce que** le fil central (130) est fixé dans la lumière (120) à distance de l'extrémité proximale (152) du fil hélicoïdal (150) dans une direction proximale et est disposé en appui sur une surface limite de la lumière (120) en particulier de manière excentrée par rapport à un axe longitudinal de la lumière.

6. Fil de guidage selon l'une des revendications 1 à 5, **caractérisé en ce qu'**un diamètre extérieur (130.1, 130.2) du fil central (130) est inférieur à un diamètre intérieur (120.1) de la lumière (120) et/ou inférieur à un diamètre intérieur (150.1) du fil hélicoïdal (150), le diamètre intérieur (120.1) de la lumière (120) et/ou le diamètre intérieur (150.1) du fil hélicoïdal (150) est 3 à 4 fois supérieur au diamètre extérieur (130.2) du fil central (130) dans la région du fil hélicoïdal (150).

7. Fil de guidage selon l'une des revendications 1 à 6, **caractérisé en ce qu'**une distance (154.1, 154.1) est ménagée entre les au moins deux enroulements adjacents et correspond à 0,1-0,5 fois le diamètre de fil du fil hélicoïdal (150).

8. Fil de guidage selon l'une des revendications 1 à 7, **caractérisé en ce qu'**un diamètre intérieur (150.1) du fil hélicoïdal (150) est sensiblement constant sur toute la longueur et correspond approximativement au diamètre intérieur de la tige creuse (110) ou au diamètre de la lumière cylindrique creuse (120).

9. Fil de guidage selon la revendication 8, **caractérisé en ce qu'**une pluralité d'ouvertures de passage (354a, 354b, 354c, 354d) et une pluralité de portions (355a, 355b, 355c, 355d) étanches aux fluides sont présentes qui sont disposées de préférence selon une séquence alternée et en particulier à des intervalles réguliers sur toute la longueur du fil hélicoïdal (350).

10. Fil de guidage selon l'une des revendications 1 à 9, **caractérisé en ce qu'**une élasticité du fil hélicoïdal (150) est supérieure à une élasticité de la tige creuse (110) .

11. Fil de guidage selon l'une des revendications 1 à 10, **caractérisé en ce que** la tige creuse (110) se présente sous la forme d'un tube en acier et le fil hélicoïdal (150) comprend notamment un fil de platine.

12. Fil de guidage selon l'une des revendications 1 à 11, **caractérisé en ce que** la tige creuse (110) est formée d'au moins deux parties, une portion proximale (111) de la tige creuse (110) présentant une élasticité inférieure à celle d'une portion distale (115) de la tige creuse (110) .

13. Fil de guidage selon la revendication 12, **caractérisé en ce que** la portion proximale (111) comprend un tube métallique, notamment un tube en acier, et la portion distale (115) comprend un tube en matière synthétique, notamment un tube en polyimide.

14. Fil de guidage selon l'une des revendications 12 et 13, **caractérisé en ce que** la portion distale (115) de la tige creuse présente moins de souplesse que le fil hélicoïdal (150).

15. Fil de guidage selon l'une des revendications 1 à 14, **caractérisé en ce que** le diamètre extérieur de l'ajutage tubulaire (480) correspond approximativement au diamètre intérieur du fil hélicoïdal (450) et de préférence le diamètre intérieur de l'ajutage tubulaire (480) est supérieur au diamètre extérieur du fil central (430) dans la région de l'ajutage tubulaire (480).

16. Fil de guidage selon l'une des revendications 1 à 15, **caractérisé en ce que** le diamètre du fil central (130) diminue d'une extrémité proximale (131) à une extrémité distale (132), de préférence de telle sorte que la flexibilité du fil de guidage (130) diminue de façon continue de l'extrémité proximale (131) du fil central (130) jusqu'à l'extrémité du fil de guidage (170).

17. Fil de guidage selon l'une des revendications 1 à 16, **caractérisé en ce que** la pointe de fil de guidage (170) est réalisée à partir d'une matière synthétique.

18. Fil de guidage selon l'une des revendications 1 à 17, **caractérisé en ce que** le fil hélicoïdal comprend un seul fil et **en ce que** la région du fil hélicoïdal est exempte d'une membrane supplémentaire.

19. Fil de guidage selon l'une des revendications 12 à 18, **caractérisé en ce qu'**exactement trois parties tubulaires différentes sont pourvues d'un diamètre extérieur sensiblement égal, à savoir comme première partie tubulaire de la portion proximale (111) de la tige creuse (110), comme deuxième partie tubulaire du fil hélicoïdal (150) et comme troisième partie tubulaire d'un tube intermédiaire reliant les première et deuxième parties.

20. Fil de guidage selon l'une des revendications 1 à 19, **caractérisé en ce que** l'extrémité proximale du fil hélicoïdal (150) est ancrée dans l'extrémité distale (117) de la portion distale (115) de la tige creuse.
